# EUROPÄISCHE PATENTSCHRIFT

(11) **EP 4 311 498 B1**
(45) Veröffentlichungstag und Bekanntmachung des Hinweises auf die Patenterteilung: **14.01.2026**
(21) Anmeldenummer: 23163770.3
(22) Anmeldetag: 23.03.2023
(51) Int. Cl.: A61B 6/03, A61B 6/00

(54) **COMPUTERTOMOGRAPHIE-ANLAGE MIT EINEM KABELFÜHRUNGSYSTEM**
COMPUTER TOMOGRAPHY SYSTEM WITH CABLE GUIDE
SYSTÈME DE TOMOGRAPHIE ASSISTÉE PAR ORDINATEUR AVEC GUIDE-CÂBLE

(30) Priorität: 29.07.2022 EP 22187855
(43) Veröffentlichungstag der Anmeldung: 31.01.2024
(73) Patentinhaber: Siemens Healthineers AG, 91301 Forchheim (DE)
(72) Erfinder: Gross, Stefan, 92724 Trabitz (DE); Hupfauf, Matthias, 92507 Nabburg (DE)
(74) Vertreter: Siemens Healthineers Patent Attorneys

(56) Entgegenhaltungen:
- DE-A1- 102008 035 196
- DE-A1- 102018 219 541
- DE-A1- 102021 202 983
- JP-A- H09 220 223
- US-A1- 2019 090 830
- US-B1- 6 431 751

## Beschreibung

*Unabhängig vom grammatikalischen Geschlecht eines bestimmten Begriffes sind Personen mit männlicher, weiblicher oder anderer Geschlechteridentität mit umfasst.*

Vorliegend wird ein Kabelführungssystem für eine bewegliche Computertomographie-Anlage beschrieben, welches zumindest teilweise deckengeführt bzw. oberhalb der Gantry der Computertomographie-Anlage verläuft. Es wird weiter eine Computertomographie-Anlage mit einem derartigen Kabelführungssystem beschrieben.

In modernen medizinischen Untersuchungs- und Behandlungseinrichtungen kommen vermehrt bewegliche Computertomographie (CT)-Anlagen zum Einsatz. Die Bewegbarkeit der Anlagen dient vorwiegend dem Zweck, die typischerweise großen und Raum einnehmenden CT-Anlagen zu verstellen, um in unmittelbarer Umgebung des Patienten Platz für medizinisches Personal und/oder weitere Anlagen oder Geräte, die bei einer Untersuchung, einer Behandlung und/oder einer Intervention zum Einsatz kommen, bereit zu stellen. Dabei steht das Wohl und die Sicherheit des Patienten, aber auch des Bedienpersonals und der Maschinen im Vordergrund.

Daneben bietet eine Bewegbarkeit der CT-Anlagen auch die Möglichkeit, sie in verschiedenen Behandlungsräumen einsetzen zu können und so langfristig Investitions- und Haltungskosten zu reduzieren.

Bekannt ist es, CT-Anlagen entlang von Schienen zu verstellen, sodass die CT-Anlagen entlang der Schienen vordefinierte Positionen einnehmen können. Alternativ dazu sind auch frei bewegliche CT-Anlagen am Markt verfügbar. Während frei bewegliche CT-Anlagen über eine wiederaufladbare On-Board EnergieVersorgung, bspw. in Form eines Lithium-Ionen-Speichers, verfügen, besteht bei schienengebundenen Systemen die Herausforderung, die Versorgung leitungsgebunden zu realisieren.

Bei CT-Anlagen, die in verschiedenen, typischerweise zwei, Behandlungsräumen eingesetzt werden, müssen Kabelführungssysteme beweglich und flexibel ausgebildet sein, und zwar so, dass sie Strecken von bis zu 12 m überbrücken können. Die mechanische Beanspruchung darf jedoch die Lebensdauer des Kabelführungssystems nicht beeinträchtigen.

Zudem muss sichergestellt werden, dass das Kabelführungssystem selbst bei einer Verstellbewegung der CT-Anlage keine Kollisionen mit dem Patienten, medizinischem Personal bzw. umgebenden Geräten verursachen kann.

Entsprechend sind im Stand der Technik Lösungen für Kabelführungen vorgesehen, die im Boden, typischerweise in der Nähe des Schienensystems angeordnet sind. Hier sind Kollisionen weitgehend ausgeschlossen. Diese Lösungen erfordern jedoch bauliche Voraussetzungen der Krankenhausumgebung und sind daher nicht uneingeschränkt einsetzbar. Zudem sind sie oft nicht auf die Hygiene-Anforderungen einer medizinischen Umgebung angepasst und teuer.

Alternativ sind Lösungen bekannt, bei denen Versorgungsleitungen mittels einer oder mehrerer Energieführungsketten in einem Deckenkasten angeordnet werden. Das Schienensystem verläuft hier parallel zur Längsachse des Deckenkastens. An der Gantry wird eine mitbewegte Vertikalsäule vorgesehen, durch die die Versorgungsleitungen nach unten zum Fuß der Gantry geführt und dort angeschlossen werden. Die Länge des Deckenkastens definiert dabei den maximalen Verstellweg für die Gantry.

Aus der Druckschrift US 2019 090 830 A1 ist ein Bildgebungssystem bekannt. Das Bildgebungssystem umfasst ein erstes Bildgebungsgerät und eine zweite Gantry. Das erste Bildgebungsgerät umfasst eine erste Gantry, eine erste Röntgenquelle, die auf der ersten Gantry bereitgestellt ist, und einen ersten Detektor, der der ersten Röntgenquelle gegenüberliegt und auf der ersten Gantry bereitgestellt ist. Das erste Bildgebungsgerät wird verwendet, um einen ersten Bildgebungsmodus auszuführen. Die zweite Gantry ist trennbar mit der ersten Gantry verbunden und führt einen zweiten kombinierten Bildgebungsmodus aus.

Die Patentschrift US 64 317 51 B1 beschreibt ein diagnostisches Bildgebungssystem mit einem C-Arm zur Unterstützung einer Röntgenquelle und eines Flachbilddetektors. Der C-Arm enthält weiterhin einen Kuppler, der es dem C-Arm ermöglicht, an verschiedene deckenmontierte Stützstrukturen und an einen mobilen Wagen für mobile C-Arm-Bildgebungsanwendungen abnehmbar befestigt zu werden. Ein Transportwagen wird bereitgestellt, um den C-Arm von einem ersten Operationssaal in einen zweiten Operationssaal zu transportieren. Ein mobiler Gerätewagen kann den Transportwagen begleiten. Alternativ kann eine zentrale Steuereinrichtung Befehls- und Steuersignale für den C-Arm bereitstellen.

Demgegenüber ist es Aufgabe der vorliegenden Erfindung, alternative Mittel für eine Kabelführung für eine bewegliche CT-Anlage bereit zu stellen, die bei hoher Lebensdauer und geringen Baukosten eine höhere Bewegungsflexibilität ermöglichen. Insbesondere ist es Aufgabe der vorliegenden Erfindung, die Bewegungsfreiheit kabel- und schienengebundener CT-Anlagen so weit zu erhöhen, dass sie in verschiedenen Behandlungsräumen mit entgegengesetzter Betriebsrichtung eingesetzt werden können. Ferner ist es Aufgabe der vorliegenden Erfindung, den Footprint einer beweglichen Computertomographie-Anlage weiter zu verringern.

Diese Aufgabe wird gelöst durch Computertomographie-Anlage, umfassend ein Kabelführungssystem, gemäß Anspruch 1.

Das Kabelführungssystem gemäß einem ersten Aspekt der Erfindung dient der Versorgung, insbesondere der energetischen Versorgung der Computertomographie-Anlage. Entsprechend handelt es sich bei einer Versorgungsleitung um ein ElektroKabel. Alternativ oder zusätzlich kann die Versorgungsleitung der Datenkommunikation von bzw. zu der Computertomographie-Anlage dienen. Daten liegen bspw. in Form von Rohdaten oder bereits rekonstruierten Bilddaten vor. Daten können auch Steuerdaten betreffend den Betrieb der Computertomographie-Anlage umfassen. Insofern kann die Versorgungsleitung auch oder alternativ als Datenkabel ausgebildet sein. Das Kabelführungssystem ist ausgebildet eine oder mehrere Versorgungsleitungen zu führen bzw. aufzunehmen. Eine Versorgungsleitung kann in Ausführungen bis zu 40 Kabel bzw. Leitungen umfassen. Die Gantry der Computertomographie-Anlage ist verstellbar ausgebildet, und zwar in einer zur Gantry senkrecht verlaufenden Bewegungsrichtung. Die Gantry weist eine zentrale Öffnung, die Bore, auf, in welcher ein Patient für eine Bildgebung zumindest teilweise positioniert werden kann. Die Patientenlängsachse entspricht erfindungsgemäß der Bewegungsrichtung der Gantry.

Insofern wird im Folgenden ohne Beschränkung der Allgemeinheit von einem Menschen als Patienten ausgegangen. Grundsätzlich kann der Patient auch ein Tier sein.

Das Kabelführungssystem umfasst eine an der Gantry angeordnete Vertikalsäule, die von einem Sockel der Computertomographie-Anlage vertikal nach oben verläuft. Die Vertikalsäule endet bodenseitig auf bzw. mit dem Sockel der Gantry und wird folglich mit der Gantry mitbewegt, wenn diese in der Bewegungsrichtung verschoben wird. Die Vertikalsäule weist eine Höhe auf, die größer als die Höhe der Gantry inklusive Sockel ist. Die Vertikalsäule überragt also die Gantry höhenmäßig. Auch die Vertikalsäule ist so dimensioniert, dass sie wenigstens eine Versorgungsleitung, bevorzugt eine Mehrzahl von Versorgungsleitungen in sich aufnehmen bzw. in sich führen kann. Die Vertikalsäule besteht in Ausführungen aus einen AluminiumBlech oder aus einem Kunststoff.

Das Kabelführungssystem umfasst weiter einen ersten Gelenkarm sowie einen zweiten Gelenkarm. Der erste Gelenkarm ist an dem oberen, also deckengerichteten Ende der Vertikalsäule oberhalb der Gantry über einen ersten Gelenkpunkt rotierbar angebunden. Mit anderen Worten kann der erste Gelenkarm seine Relativposition zu der Gantry über eine Rotation bzw. über ein Verschwenken um den ersten Gelenkpunkt ändern. Der erste Gelenkarm ist weiter mit dem ebenfalls oberhalb der Gantry angeordneten zweiten Gelenkarm über einen zweiten Gelenkpunkt rotierbar angebunden. Mit anderen Worten kann der erste Gelenkarm auch seine Relativposition zu dem zweiten Gelenkarm über eine Rotation bzw. über ein Verschwenken um den zweiten Gelenkpunkt ändern.

Sowohl die Vertikalsäule als auch der erste und der zweite Gelenkarm sind ausgebildet, wenigstens eine Versorgungsleitung, bevorzugt eine Vielzahl von Versorgungsleitungen, die dann parallel zueinander bzw. nebeneinander angeordnet sind, zumindest teilweise jeweils entlang ihrer Längsachsen zu führen. Wie eingangs mit Bezug zu der Vertikalsäule schon erläutert, bilden die einzelnen Komponenten des Kabelführungssystems jeweils Hohlräume bzw. zumindest teilweise von außen abgeschirmte Innenbereiche aus, die sich jeweils in Längsrichtung der Komponenten erstrecken und in oder an welchen die wenigstens eine Versorgungsleitung aufgenommen, angeordnet oder integriert werden kann. Durch den ersten und den zweiten Gelenkpunkt sowie den ersten Gelenkarm ist die Bewegung der Gantry der CT-Anlage vom zweiten Gelenkarm entkoppelt, was eine größere Flexibilität im Hinblick auf die Positionierbarkeit der Gantry bewirkt.

Konkret verläuft die wenigstens eine Versorgungsleitung entlang der Längsachsen des ersten und des zweiten Gelenkarms verstellbar geführt. Das heißt, dass die Versorgungsleitung einerseits parallel bzw. im Wesentlichen parallel zu den Längsachsen der Gelenkarme und der Vertikalsäule geführt wird bzw. verläuft. Andererseits heißt das, dass die Versorgungsleitung relativ zu den Gelenkarmen entlang ihrer Längsachse verstellt bzw. verschoben werden kann. Mit anderen Worten kann die Relativposition der Versorgungsleitung zu den beiden Gelenkarmen verändert werden. Insbesondere ist die Versorgungsleitung in Ausführungen der Erfindung ausgebildet, entlang der Gelenkarme zu gleiten. In Bezug auf die Vertikalsäule ist die Relativposition der Versorgungsleitung bevorzugt fest bzw. unveränderlich ausgebildet.

Die beiden Gelenkarme werden in Ausführungen der Erfindung aus Stahlblech hergestellt, wobei der Herstellungsprozess insbesondere ein Lasern und ein Biegen umfasst. Die Herstellung muss aber nicht auf diese beiden Schritte beschränkt sein. Damit sind die Gelenkarme vergleichsweise kostengünstig in der Herstellung und weisen ein geringes Gewicht auf, was die Anforderungen an die Auslegung der Antriebseinheiten reduziert.

In bevorzugter Ausführung des erfindungsgemäßen Kabelführungssystems ist an dem ersten und/oder dem zweiten Gelenkarm eine erste und/oder eine zweite Umlenkrolle vorgesehen, um welche die Versorgungsleitung umfänglich verstellbar geführt verläuft. Bevorzugt ist mehr als eine Umlenkrolle vorgesehen. Die Versorgungsleitung verläuft erfindungsgemäß umfänglich an bzw. um eine Mantelfläche der Umlenkrolle. Die wenigstens eine Umlenkrolle ist bevorzugt an einem der Gelenkpunkte angeordnet. Die Umlenkrolle ist um ihre Rotationsachse, die sich parallel zu ihrer Mantelfläche erstreckt, rotierbar. Wird das Kabelführungssystem aufgrund einer Verstellbewegung der Gantry der Computertomographie-Anlage verstellt, verschiebt sich die Versorgungsleitung der Länge nach entlang der Gelenkarme. Durch das Anliegen der Versorgungsleitung an der Umlenkrolle wird diese durch die Bewegung der Versorgungsleitung rotiert und sorgt so für ein reibungsarmes Umlenken der Versorgungsleitung. Die Erfinder haben erkannt, dass anhand der wenigstens einen Umlenkrolle in wenigstens einem der Gelenkpunkte Reserveschlaufen an den Gelenkpunkten vorteilhaft vermieden werden können, ohne die Beweglichkeit des Kabelführungssystems einschränken zu müssen.

In Ausführung der Erfindung ist in dem ersten Gelenkpunkt ein Zylindersegment angeordnet, um welches die Versorgungsleitung umfänglich verstellbar geführt verläuft. Das Zylindersegment weist in bevorzugter Ausführung eine im wesentlichen Halbkreis-förmige Grundfläche auf. In besonders bevorzugter Ausführung ist die Grundfläche sogar etwas größer als ein Halbkreis ausgebildet. Mit seiner ebenen Schnittfläche ist das Zylindersegment in Ausführung der Erfindung an der Vertikalsäule befestigt, insbesondere neben oder nahe einem ebenfalls an der Vertikalsäule vorgesehenen Anbindungs- bzw. Befestigungspunkt für die Versorgungsleitung. Bis zu diesem Punkt ist die Relativposition zwischen Versorgungsleitung und Vertikalsäule festgelegt. Die erfindungsgemäße Anordnung des Zylindersegments bewirkt, dass die Versorgungsleitung zumindest teilweise in Abhängigkeit der Stellung zwischen Vertikalsäule und erstem Gelenkarm umfänglich an der Mantelfläche des Zylindersegments verläuft bzw. dort anliegt. Je nach Relativstellung zwischen erstem Gelenkarm und Gantry liegt die Versorgungsleitung also mehr oder weniger an der Mantelfläche des Zylindersegments an. Das Zylindersegment bewirkt vorteilhaft eine Führung der Versorgungsleitung direkt hinter dem Anbindungspunkt.

Die verstellbare Führung bedeutet im Zusammenhang mit dem Zylindersegment nicht eine Verschiebung der Versorgungsleitung ihrer Länge nach gegenüber dem Zylindersegment, sondern eine Anlagerung bzw. ein Entfernen der Versorgungsleitung an bzw. von der Mantelfläche des Zylindersegments. Mit anderen Worten verändert sich je nach Relativstellung zwischen Vertikalsäule und erstem Gelenkarm die Wegstrecke, die die Versorgungsleitung umfänglich an dem Zylindersegment anliegt.

Entsprechend einer besonders bevorzugten Ausgestaltung des erfindungsgemäßen Kabelführungssystems ist die erste Umlenkrolle an dem zweiten Gelenkpunkt angeordnet. Mit anderen Worten sorgt die erste Umlenkrolle in dieser Ausführung zwischen erstem und zweitem Gelenkarm für eine optimale Umlenkung der Versorgungsleitung für eine Vielzahl von Relativpositionen zwischen erstem und zweitem Gelenkarm, also für eine Vielzahl von Winkelstellungen zwischen den beiden Gelenkarmen, und das ohne, dass in dem zweiten Gelenkpunkt eine Reserveschlaufe erforderlich wäre.

In bevorzugter Ausführung des Kabelführungssystems ist der zweite Gelenkarm weiter über einen deckenständigen dritten Gelenkpunkt oberhalb der Gantry rotierbar angebunden. Der dritte Gelenkpunkt ist folglich nahe bzw. an einer Raumdecke einer Untersuchungs- bzw. Behandlungsumgebung angeordnet. Der dritte Gelenkpunkt kann in Ausführungen ortsfest an der Decke positioniert sein. In alternativen Ausführungen ist auch die Position des dritten Gelenkpunktes verstellbar, vorzugsweise in einer Richtung parallel zur Bewegungsrichtung der Gantry verstellbar ausgebildet, wie weiter unten noch genauer erläutert wird.

Der zweite Gelenkarm ist also mittel- oder unmittelbar mit der Raumdecke über den dritten Gelenkpunkt rotierbar angebunden. Mit anderen Worten kann der zweite Gelenkarm seine Relativposition zu der Raumdecke über eine Rotation bzw. über ein Verschwenken um den dritten Gelenkpunkt ändern.

Durch das Vorsehen eines dritten Gelenkpunktes am zweiten Gelenkarm kann die Positionierungsfreiheit für die CT-Gantry weiter erhöht werden. Über den ersten und den zweiten Gelenkarm, die jeweils gegeneinander bzw. gegen die Gantry bzw. die Raumdecke verschwenkt werden können, ist die Bewegung der Gantry maximal von der Raumdecke entkoppelt. Insbesondere ermöglicht diese Entkopplung eine Rotation der Gantry um eine Vertikalachse, die durch das Iso-Zentrum der Gantry verläuft.

In weiterer bevorzugter Ausführung ist in dem dritten Gelenkpunkt die zweite Umlenkrolle angeordnet, um welche die Versorgungsleitung umfänglich verstellbar geführt verläuft. Mit anderen Worten sorgt die zweite Umlenkrolle in dieser Ausführung zwischen erstem und zweitem Gelenkarm für eine optimale Umlenkung der Versorgungsleitung für eine Vielzahl von Relativpositionen zwischen zweitem Gelenkarm und der Raumdecke bzw. einer vorab definierten Raumdeckenachse, also für eine Vielzahl von Winkelstellungen zwischen zweitem Gelenkarm und Raumdeckenachse. Dies gilt auch hier ohne, dass eine Reserveschlaufe in dem dritten Gelenkpunkt erforderlich wäre.

In Bezug auf die erste und/oder die zweite Umlenkrolle bedeutet die umfänglich verstellbare Führung, dass einerseits die Versorgungsleitung entlang ihrer Längsachse relativ zu der Umlenkrolle verstellt werden kann. Die umfänglich verstellbare Führung adressiert aber auch die umfängliche Wegstrecke um die jeweilige Umlenkrolle herum, die in Abhängigkeit der Relativstellung von erstem und zweitem Gelenkarm bzw. zweitem Gelenkarm und drittem Gelenkpunkt unterschiedlich lang ausfallen kann. Je größer bspw. der Winkel zwischen erstem und zweitem Gelenkpunkt ist, umso kleiner ist die umfängliche Wegstrecke, entlang derer die Versorgungsleitung an der ersten Umlenkrolle anliegt.

Die optimale Umlenkung meint erfindungsgemäß insbesondere, dass die erste und/oder die zweite Umlenkrolle derart ausgelegt bzw. dimensioniert sind, dass für eine Vielzahl von Stellungen der Gelenkarme zueinander bzw. zu der Gantry bzw. zu dem deckengehängten dritten Gelenkpunkt die Länge der Versorgungsleitung zwischen Anbindungspunkt an der Vertikalsäule und dem dritten Gelenkpunkt immer gleich ist. Die Vielzahl von Stellungen des Kabelführungssystems umfasst insbesondere alle Stellungen, die bei einer reinen Translation der Gantry zwischen zwei maximal entfernten Verstellpositionen der Gantry eingenommen werden können. Das bedeutet, dass für eine reine Translation der Gantry erfindungsgemäß die dafür erforderliche Reservelänge für die Versorgungsleitung allein über die variablen an den Umlenkrollen umfänglich vorgesehenen Verstellwege vorgehalten bzw. ausgeglichen wird. Anders ausgedrückt ist bei einer reinen Längsfahrt der Gantry die Länge der Versorgungsleitung zwischen erstem und dritten Gelenkpunkt konstant. Die variablen Verstellwege an erstem, zweiten und dritten Gelenkpunkt gleichen sich für jede Stellung der Gantry aus, solange diese nur translatiert.

Für eine Rotation der Gantry insbesondere um 180° ist in Ausführungen der Erfindung eine weitere Versorgungsleitungsreserve vorgesehen, wie weiter unten noch näher beschrieben wird.

Die wenigstens eine Versorgungsleitung ist in Ausführung der Erfindung in einer Energiekette geführt, dabei ist die Versorgungsleitung besonders bevorzugt über die gesamte Länge der Versorgungsleitung vorgesehen. Die Energiekette wird bei einer relativen Verstellbewegung der Versorgungsleitung ggü. weiteren Komponenten des Kabelführungssystem mit dieser entlang ihrer Längsachse mitbewegt. Die Energiekette bildet einen mechanischen Schutz für die wenigstens eine Versorgungsleitung. Insbesondere an den Gelenkpunkten verläuft die wenigstens eine Versorgungsleitung zumindest teilweise ohne weiteren äußeren Schutz, sodass die Energiekette insbesondere dort Beschädigungen der Versorgungsleitung verhindert. Zudem kann die Außenseite der Versorgungsleitung über ihre gesamte Länge im Hinblick auf ihr Gleitverhalten optimiert ausgebildet sein, um die Relativbewegung zwischen der Energiekette und weiteren Komponenten des Kabelführungssystems zu erleichtern. Entsprechend kann die Oberfläche in Ausführungen der Erfindung besonders reibungsarm ausgeführt sein. Die Energiekette verhindert auch ein übermäßiges, zu Beschädigungen führendes Verbiegen oder Knicken der Versorgungsleitung.

Der zweite Gelenkpunkt des erfindungsgemäßen Kabelführungssystems ist bevorzugt derart ausgebildet, dass er Stellungen erlaubt, in denen der erste Gelenkarm und der zweite Gelenkarm einen Winkel zwischen 10° und 170° einschließen. Die Winkelangabe bezieht sich hierbei auf die beiden Längsachsen der Gelenkarme. Mit anderen Worten erlaubt der zweite Gelenkpunkt Ausrichtungen von erstem und zweitem Gelenkarm, in denen diese fast bzw. im Wesentlichen parallel entweder hintereinander oder nebeneinander angeordnet sind. Die erste Ausrichtung ermöglicht einen vorteilhaft großen Abstand zwischen Vertikalsäule und dem deckengehängten dritten Gelenkpunkt, was die Bewegungsfreiheit für die Gantry erhöht. Die zweitgenannte Ausrichtung realisiert hingegen einen minimalen Footprint der gesamten CT-Anlage. Mit anderen Worten benötigt die CT-Anlage mit dieser Ausrichtung der beiden Gelenkarme einen kleinstmöglichen Grundflächen- bzw. Raumbedarf. Dies ermöglicht vorteilhaft eine kleinere Dimensionierung von Parkflächen für die CT-Anlage, wenn diese außer Betrieb ist.

Die Rotationachse der ersten Umlenkrolle verläuft in bevorzugter Ausführung des Kabelführungssystems durch den zweiten Gelenkpunkt und der erste und der zweite Gelenkarm sind auf der Rotationsachse der ersten Umlenkrolle aneinander angebunden. Derart definiert der Durchmesser der ersten Umlenkrolle im Wesentlichen einen Mindestabstand der beiden Gelenkarme. Dieser Mindestabstand wird mittels sowohl am ersten als auch am zweiten Gelenkarm vorgesehener Hebelarme überbrückt, die sich bspw. in einem Winkel von ca. 30° bis 120° ggü. der Gelenkarmlängsachse an dem dem ersten Gelenkpunkt zugerichteten Ende vom Gelenkarm weg erstrecken. Besonders bevorzugt umfasst jeder Gelenkarm zwei Hebelarme an dem dem ersten Gelenkpunkt zugerichteten Ende, wobei die freistehenden Enden der Hebelarme jeweils mit einer entsprechenden Öffnung versehen sind und bspw. mittels eines die Öffnungen durchsetzenden Bolzens auf der Rotationachse der ersten Umlenkrolle gehalten werden. Die Hebelarme umgreifen die erste Umlenkrolle dabei bevorzugt sowohl oben als auch unten. Andere Gestaltungsvarianten zur Anbindung des ersten und des zweiten Gelenkarms sind ebenfalls denkbar und im Sinne der Erfindung. In Ausführungen können die Hebelarme die effektive Länge eines Gelenkarms erhöhen.

In weiterer Ausführung des erfindungsgemäßen Kabelführungssystems ist der erste Gelenkpunkt derart ausgebildet, dass er Stellungen erlaubt, in denen der erste Gelenkarm und die Gantry einen Winkel zwischen 10° und 180° einschließen. Die Winkelangabe bezieht sich hierbei auf die Längsachse des ersten Gelenkarms sowie eine vorab zu definierende Querachse der Gantry bzw. der Vertikalsäule.

Mittels der besonders breit vorgegebenen Rotationswinkelbereiche des ersten und des zweiten Gelenkpunktes ermöglicht das Kabelführungssystem insbesondere eine Rotation der Gantry um 180° und darüber hinaus.

In weiterer bevorzugter Ausführung des Kabelführungssystems verläuft die Mittelachse des Zylindersegments durch den ersten Gelenkpunkt und der erste Gelenkarm ist in bzw. auf der Mittelachse an der Vertikalsäule angebunden.

Derart definiert der Radius des Zylindersegments im Wesentlichen einen Mindestabstand zwischen der Längsachse des ersten Gelenkarms und der Querachse der Vertikalsäule. Dieser Mindestabstand wird mittels wenigstens eines, bevorzugt zweier am ersten Gelenkarm vorgesehener Hebelarme überbrückt, die sich ebenfalls in einem Winkel von ca. 30° bis 120° ggü. der Gelenkarmlängsachse an dem der Vertikalsäule zugerichteten Ende vom ersten Gelenkarm weg erstrecken. Bevorzugt umfasst der erste Gelenkarm auch hier zwei Hebelarme, wobei die freistehenden Enden der Hebelarme wieder jeweils mit einer entsprechenden Öffnung ausgebildet sind und bspw. mittels eines die Öffnungen durchsetzenden Bolzens auf der Mittelachse des Zylindersegments gehalten werden. Die Hebelarme umgreifen das Zylindersegment wieder bevorzugt sowohl oben als auch unten. Der Vollständigkeit halber sei angemerkt, dass das Zylindersegment fest an der Vertikalsäule montiert, also nicht ausgebildet ist, um seine Mittelachse zu rotieren. In Ausführungen können die Hebelarme auch hier die effektive Länge des ersten Gelenkarms erhöhen.

In weiterer Ausführung des Kabelführungssystems ist der dritte Gelenkpunkt ausgebildet, dass er Stellungen erlaubt, in denen der zweite Gelenkarm und eine vorab zu definierende Achse an der Raumdecke, insbesondere eine Achse parallel zu einer translatorischen Verstellrichtung der Gantry, bspw. der Längsachse der weiter unten noch näher beschriebenen Horizontalsäule, entlang derer sich ein Laufwagen bewegt, einen Winkel zwischen 10° und 270° einschließen. Dadurch wird die Bewegungsfreiheit der CT-Anlage weiter erhöht, da somit auch der zweite Gelenkarm ausgehend vom dritten Gelenkpunkt einen breiten Winkelbereich überspannen kann. In besonderen Ausführungen kann durch den besonders weit gefassten Winkelbereich des dritten Gelenkpunktes die Auslegung entweder der Gelenkarmlängen und oder der durch die weiter unten beschriebene Horizontalsäule bereitgestellte translatorische Verstellweg kürzer sein, ohne den Bewegungsradius für die Gantry insbesondere im Hinblick auf ihre isozentrische Rotation einschränken zu müssen. Derart kann unter Materialeinsparung das Kabelführungssystem besonders gut an kleinere Untersuchungsumgebungen angepasst werden.

In Ausführungen des Kabelführungssystems verlaufen bzw. liegen bzw. bewegen sich der erste und der zweite Gelenkarm in einer Ebene. Das heißt, der erste, der zweite und auch der dritte Gelenkpunkt ermöglichen jeweils eine Rotation bzw. ein Verschwenken um parallel zueinander angeordnete Rotationsachsen. Entsprechend einer bevorzugten Ausführungsvariante liegen die beiden Gelenkarme in einer Horizontalebene, sind also parallel zu einem Untergrund oder der Raumdecke angeordnet. Die Gelenkpunkte können entsprechend besonders einfach ausgebildet sein, da sie bspw. wie oben bereits ausgeführt, jeweils nur einen Bewegungsfreiheitsgrad für die Verschwenkbewegung bereitstellen müssen.

Die Vertikalsäule kann aber auch höhenverstellbar ausgebildet sein. Dann können in Ausführungen der erste, zweite und/oder dritte Gelenkpunkt ausgebildet sein, auch eine Verschwenkbewegung um eine Horizontalachse zu erlauben. In diesem Fall kann die Höhe der Vertikalsäule insbesondere nachträglich, also nach einer Installation der CT-Anlage leicht angepasst werden, um bspw. räumlichen Vorgaben einer spezifischen Untersuchungs- oder Behandlungsumgebung gerecht zu werden. In anderen Ausführungen wird die Höhe der Vertikalsäule bereits vor einer Montage bspw. auf eine vorherrschende Raumhöhe eingestellt. Es ist dann ausreichend, dass erster, zweiter und/oder dritter Gelenkpunkt jeweils nur einen Freiheitsgrad aufweisen. Die Höhenverstellbarkeit der Vertikalsäule kann bspw. über eine Teleskop-Ausführung realisiert werden. Dabei umfasst die Vertikalsäule bspw. zwei oder mehr HohlProfilsegmente, wovon jeweils zwei zumindest teilweise ineinander geführt gelagert sind. Durch Ausfahren der einzelnen Hohlprofilsegmente aus dem jeweils tragenden Segment kann folglich eine Längung der Vertikalsäule erreicht werden. Eine Verkürzung kann entsprechend umgekehrt durch Einfahren der Profilsegmente ineinander erzielt werden.

Die Höhenverstellbarkeit der Vertikalsäule dient vorrangig einer Anpassung des Kabelführungssystems an bestehende räumliche Vorgaben, insbesondere eine Raumdeckenhöhe, also einem Ausgleich zwischen der Höhe des dritten Gelenkpunktes und der Vertikalsäulenhöhe.

Die Längen des ersten und des zweiten Gelenkarms werden erfindungsgemäß so gewählt, dass die Länge des ersten Gelenkarms 65% bis 75%, insbesondere 68& bis 72%, besonders bevorzugt 70% der Länge des zweiten Gelenkarms entspricht. Empirisch konnten die Erfinder feststellen, dass dieses Längenverhältnis der Gelenkarme eine Bewegungsfreiheit der Gantry besonders gut unterstützt. Insbesondere unterstützt dieses Längenverhältnis der Gelenkarme eine rein rotatorische Bewegung der Gantry um eine Vertikalachse durch ihr Isozentrum um 180°.

In besonders bevorzugter Ausführung ist der erste Gelenkarm maximal 1600 mm und der zweite Gelenkarm maximal 2300 mm lang ist. Mit den angegebenen maximalen Längen der Gelenkarme lässt sich mit dem bislang beschriebenen Kabelführungssystem bereits eine maximale Verfahrstrecke für die Gantry von 5600 mm erreichen. Die Längen der beiden Gelenkarme können in Ausführungen auch entsprechend dem obigen Verhältnis verkürzt werden, um das Kabelführungssystem an bauliche Gegebenheiten der Untersuchungs- oder Behandlungsumgebung oder die Dimensionen der CT-Anlage anzupassen.

Das Kabelführungssystem umfasst ferner in bevorzugter Ausführung eine oberhalb der Gantry verlaufende, deckenständige Horizontalsäule, die sich in ihrer Längsachse parallel zu der translatorischen Bewegungsrichtung der Gantry erstreckt. Dabei ist an der Horizontalsäule ein in Längsrichtung der Horizontalsäule verstellbarer Laufwagen angeordnet, der den dritten Gelenkpunkt trägt. Der dritte Gelenkpunkt, der in anderen Ausführungen fest an der Raumdecke positioniert sein kann, lässt sich in dieser Ausführung parallel zur Bewegungsrichtung der Gantry verstellen. Vorteilhaft lässt sich der Laufwagen dabei über im Wesentlichen die gesamte Länge der ebenfalls deckenständigen Horizontalsäule verstellen. Derart verlängert sich die maximale Verfahrstrecke der Gantry mit den maximalen Längen des ersten und zweiten Gelenkarms auf 12 m und ermöglicht dabei komfortabel einen Einsatz der Computertomographie-Anlage in mehreren Behandlungsräumen.

Alternativ oder zusätzlich zu einer höhenverstellbaren Vertikalsäule, wie oben bereits beschrieben, kann auch der Laufwagen ausgebildet sein, die Höhe des dritten Gelenkpunktes zu verstellen, sodass auch mittels des Laufwagens eine Anpassung des Kabelführungssystems an eine vorgegebene Raumdeckenhöhe realisierbar ist.

Die wenigstens eine Versorgungsleitung wird dann ausgehend von dem dritten Gelenkpunkt am Laufwagen wenigstens teilweise in wenigstens einer Energiekette in der Horizontalsäule weitergeführt. Die Energiekette sorgt, wie eingangs schon beschrieben, für einen mechanischen Schutz der wenigstens einen Versorgungsleitung und verhindert insbesondere ein Verknicken der Versorgungsleitung, wenn der Laufwagen zwischen einem ersten Ende der Horizontalsäule und dem zweiten Ende der Horizontalsäule entlang ihrer Längsachse verstellt wird.

In Ausführungen der Erfindung kann der Laufwagen passiv durch einen in der Gantry bzw. im Sockel der Gantry vorgesehenen Antrieb mitbewegt werden. In anderen Ausführungen kann der Laufwagen alternativ oder zusätzlich über eine eigene Antriebseinheit verfügen, um den Laufwagen aktiv entlang der Horizontalsäule zu bewegen. Dies ist insbesondere von Vorteil, um die CT-Anlage in ihre Park-Position bzw. in ihre Parkstellung zu verbringen, in der der erste und der zweite Gelenkarm bzw. einen Winkel von 10° zueinander einnehmen und im Wesentlichen nebeneinander liegend angeordnet sind.

Die Horizontalsäule weist in Ausführungen der Erfindung eine Länge von maximal 7 m entlang der Bewegungsrichtung der Gantry auf. Mit anderen Worten wird der Laufwagen bei einer Bewegung der Gantry über eine Strecke von maximal 7 m mit verstellt.

In weiteren Ausbildungen des Kabelführungssystems verläuft die Rotationachse der zweiten Umlenkrolle durch den dritten Gelenkpunkt und der zweite Gelenkarm ist auf der Rotationsachse der zweiten Umlenkrolle an dem Laufwagen angebunden. Derart definiert auch der Radius der zweiten Umlenkrolle im Wesentlichen einen Mindestabstand des zweiten Gelenkarms und des Laufwagens. Dieser Mindestabstand wird mittels am zweiten Gelenkarm vorgesehener Hebelarme überbrückt, die sich bspw. in einem Winkel von ca. 30° bis 120° ggü. der Gelenkarmlängsachse an dem dem dritten Gelenkpunkt zugerichteten Ende vom Gelenkarm weg erstrecken. Besonders bevorzugt umfasst der zweite Gelenkarm zwei Hebelarme an dem dem dritten Gelenkpunkt zugerichteten Ende, wobei die freistehenden Enden der Hebelarme wieder jeweils mit einer entsprechenden Öffnung ausgestattet sind und bspw. mittels eines die Öffnungen durchsetzenden Bolzens auf der Rotationachse der zweiten Umlenkrolle gehalten werden. Die Hebelarme umgreifen die zweite Umlenkrolle dabei bevorzugt sowohl oben als auch unten. In Ausführungen können die Hebelarme die effektive Länge des zweiten Gelenkarms erhöhen.

Besonders bevorzugt umfasst der oben eingeführte Laufwagen eines erfindungsgemäßen Kabelführungssystems ein Reservemodul zur Bereitstellung einer Versorgungsleitungsreserve. Diese Versorgungsleitungsreserve wird benötigt, sobald die Gantry isozentrisch rotiert. Während die über das Zylindersegment sowie die erste und zweite Umlenkrolle bereitgestellte Versorgungsleitungsreserve insbesondere ausreichend ist, eine Bewegung der Gantry entlang der gesamten Horizontalsäule vollständig nachzuführen, stellt die im Laufwagen vorgesehene Versorgungsleitungsreserve eine zusätzliche Versorgungsleitungslänge bereit, sodass die Gantry an jeder beliebigen Position entlang der Horizontalsäule zusätzlich um 180° um ihre eigene Achse isozentrisch rotiert werden kann.

Besonders günstig ist die Versorgungsleitungsreserve in oder dicht an dem Laufwagen angeordnet, sodass auch hier keine Leitungsschlaufe frei im Raum hängt, wodurch die Betriebssicherheit der Modalität erhöht und der Footprint vorteilhaft klein gehalten werden kann.

Das Reservemodul umfasst dabei in Ausführung der Erfindung eine Versorgungsleitungsschlaufe sowie eine dritte gegen eine Federkraft angestellte Umlenkrolle, um welche die Versorgungsleitungsschlaufe umfänglich verstellbar geführt ist. Die dritte Umlenkrolle ist also gegen die Federkraft verstellbar angeordnet bzw. montiert. Das Ende der Versorgungsleitungsreserve ist bspw. an einer tragenden Komponente des Laufwagens oder seinem Gehäuse fixiert. Wird die Versorgungsleitungsreserve bspw. für eine Rotation der Gantry benötigt, übt die Versorgungsleitung entlang ihrer Längsachse Zug auf das Reservemodul aus. Mittels dieser Zugkraft wird die dritte Umlenkrolle gegen die Federkraft in Richtung der Zugkraft verstellt, was die Versorgungsleitungsreserve zumindest teilweise frei gibt. Wird die Versorgungsleitungsreserve nicht mehr benötigt bzw. lässt der Zug auf das Reservemodul nach, bewegt die Federkraft die dritte Umlenkrolle automatisch zurück in ihre Ausgangsposition und die Versorgungsleitungsreserve wird wieder im bzw. dicht am Laufwagen aufgenommen.

Das Reservemodul ist bevorzugt derart ausgelegt, dass es eine Versorgungsleitungsreserve zwischen 45 cm und 110 cm, bevorzugt zwischen 55 cm und 65 cm, besonders bevorzugt zwischen 61 cm und 62 cm bereitstellt. Dabei ergibt sich die auszugleichende Wegstrecke als Produkt aus Drehwinkel der Gantry (maximal 180°), Pi und dem auf 180° normierten Krümmungsradius (= Radius der dritte Umlenkrolle).

In obiger bevorzugter Ausführung ist die Versorgungsleitungsreserve nach dem Flaschenzugprinzip um die dritte Umlenkrolle geführt. Das bedeutet, dass der Verstellweg der dritten Umlenkrolle ca. halb so lang ausgeführt sein muss wie die Versorgungsleitungsreserve. Der Verstellweg der dritten Umlenkrolle beläuft sich vorteilhaft auf Strecken zwischen 22,5 cm bis 40 cm, besonders bevorzugt auf Strecken zwischen 28 cm bis 33 cm.

Die Umlenkrollen des erfindungsgemäßen Kabelführungssystems sind bevorzugt alle mit denselben Maßen dimensioniert. Bevorzugt beläuft sich der Radius der Umlenkrollen auf einen Wert zwischen 18 cm bis 38 cm, wobei kleinere Radien im Hinblick auf Materialkosten, Gesamtgewicht und Footprint der Anlage günstiger sind. Die Höhe der Umlenkrollen beläuft sich dabei bevorzugt auf ca. 20 cm. Die Umlenkrollen sind bevorzugt als Kunststoffteile (bspw. Spritzgussteil) ausgebildet, wobei ein zentraler Steg entlang der Rotationsachse einer Umlenkrolle eine Grund- und eine Deckplatte auf Abstand entsprechend der Umlenkrollenhöhe hält und wobei die Umfänge der Grund- und Deckfläche gemeinsam die Mantelfläche der Umlenkrolle aufspannen. Die wenigstens eine Versorgungsleitung liegt in dieser Ausführung an diesen Umfängen der Grund- und Deckfläche an.

Die Auslegung des Zylindersegments wird im Hinblick auf Material, Höhe und Radius in bevorzugter Ausführung entsprechend den Umlenkrollen gewählt.

Um die Montage des erfindungsgemäßen Kabelführungssystems erheblich zu vereinfachen, sind der erste und der zweite Gelenkarm bevorzugt jeweils als tragendes Hohlprofil ausgebildet, wobei die wenigstens eine Versorgungsleitung entlang der Längsachsen des ersten und des zweiten Gelenkarms außerhalb des Hohlprofils in einem Halteelement verläuft. Das Halteelement ist als Bestandteil eines jeden Gelenkarms zu verstehen, wobei das Haltelement ebenfalls aus Stahlblech gefertigt werden kann. Das Halteelement kann dabei an ein Hohlprofil einstückig durch bspw. Biegen und Stanzen mit angeformt werden. Alternativ kann das Halteelement auch separat gefertigt und anschließend mit einem Hohlprofil verbunden werden. Die wenigstens eine Versorgungsleitung wiegt je nach Modalität und Ausführungen über 100 kg, welche nur unter großem Personalaufwand gehoben, gehalten, positioniert und/oder montiert werden können. Das Halteelement ist dabei bevorzugt Dachrinnen-artig bzw. Kanal-artig ausgebildet, wobei die wenigstens eine Versorgungsleitung zumindest teilweise in dem Halteelement aufgenommen wird und in diesem verläuft. Insbesondere ist das Haltelemente selbst als tragendes Element ausgeführt, welches an sich ausgebildet ist, die wenigstens eine Versorgungsleitung zu halten.

In bevorzugter Ausführung kann das Haltelement seitlich nach Integration der wenigstens einen Versorgungsleitung mittels einer Blende oder Verkleidung verschlossen werden, sodass die Versorgungsleitung vor Umgebungseinflüssen oder Staub geschützt ist. Die Computertomographie-Anlage zur Erzeugung tomographischer Röntgenbilder ist eingerichtet, tomographische Bilddaten eines Patienten, genauer einer Körperregion eines Patienten mittels Röntgenstrahlung zu erzeugen. Zu diesem Zweck umfasst die Computertomographie-Anlage in ihrer Gantry Bildgebungskomponenten in Form wenigstens einer Röntgenstrahlenquelle und wenigstens eines gegenüberliegend angeordneten Röntgenstrahlendetektors. Die Röntgenstrahlenquelle ist ausgebildet Röntgenstrahlung zu erzeugen und in Richtung Patienten, der im Isozentrum der Gantry angeordnet ist, auszusenden. Die durch die Gewebeverteilung der untersuchten bzw. abgebildeten Körperregion wird die Röntgenstrahlung abgeschwächt und trifft, nachdem sie den Patienten durchsetzt hat, auf den Röntgenstrahlendetektor auf. Die Bildgebungskomponenten der CT-Anlage sind in der Gantry rotierbar angeordnet, sodass Projektionsdaten aus einer Vielzahl von verschiedenen Winkelstellungen erzeugt werden können. Die CT-Anlage umfasst weiter eine Recheneinheit, die ausgebildet ist, aus einer Vielzahl von erfassten Projektionsdaten ein bevorzugt dreidimensionales tomographisches Röntgenbild der Körperregion zu rekonstruieren.

In Ausführungen kann die CT-Anlage ein Schienensystem umfassen, welches in der oben beschriebenen Bewegungsrichtung der Gantry verläuft. Das Schienensystem kann dabei eine, aber auch zwei oder mehr Schienen umfassen. Die Schienen sind in bevorzugter Ausführung geradlinig ausgebildet. In besonderen Ausführungen erstreckt sich das Schienensystem über eine Länge von bis zu 12 m. Diese maximale Verfahrstrecke erstreckt sich besonders vorteilhaft und über zwei Untersuchungsräume einer medizinischen Einrichtung. Bei einer maximalen Verfahrstrecke von 12 m können zwei großzügige Untersuchungsräume mit nur einer CT-Anlage genutzt werden, indem die Gantry der CT-Anlage entlang des Schienensystems verstellt wird. Dabei unterstützt das oben beschriebene Kabelführungssystem, welches in Ausführungen ausgebildet ist, über diese maximale Verfahrstrecke die wenigstens eine Versorgungsleitung mitzuführen.

In vorteilhafter Ausführung ist zwischen den beiden Untersuchungsräumen eine Park-Position vorgesehen, die mittels bspw. Schiebetüren von den Untersuchungsräumen getrennt werden kann, um die CT-Anlage außer Betrieb dort zu positionieren. Diese Parkposition kann in besonders vorteilhafter Ausführung besonders klein bzw. schmal ausgebildet sein, da die beiden Gelenkarme des Kabelführungssystems sehr dicht, fast parallel zueinander positionierbar sind.

In weiterer Ausführung kann die Horizontalsäule eine Rollobandabdeckung an der Längsseite umfassen, an welcher der Laufwagen geführt wird. Die Rollobandabdeckung dient vorwiegend einem Verschluss der Horizontalsäule. Die Rollobandabdeckung kann bspw. aus Kunststoff ausgeführt sein. Die Rollobandabdeckung kann alternativ metallisch ausgebildet sein und verringert derart Leckage-Strahlung zwischen den beiden Untersuchungsräumen.

Die CT-Anlage ist ferner ausgebildet, bei einer Verstellbewegung entlang des Schienensystems eine Rotation der Gantry um eine durch das Isozentrum der Gantry verlaufende Vertikalachse um maximal 180° zu vollziehen. Alternativ dazu kann die Gantry auch ortsfest rotiert werden. Dabei unterstützt wiederum das erfindungsgemäße Kabelführungssystem. Mittels des spezifischen Längenverhältnisses zwischen erstem und zweitem Gelenkarm kann die Gantry selbst ohne eine translatorische Bewegung entlang des Schienensystems eine Rotation um die Isozentrumsachse um 180° vollziehen.

Dabei kann die CT-Anlage einen rotatorischen Antrieb bzw. eine Drehlagerung umfassen, welcher zwischen Sockel der CT-Anlage und Gantry wirkt. Mit anderen Worten kann der Sockel ausgebildet sein, starr ausgerichtet und mit dem Schienensystem fest verbunden zu sein. Die Gantry hingegen kann durch den rotatorischen Antrieb gegenüber dem Sockel verdreht werden.

In Folgenden werden die Vorteile der Erfindung nochmal zusammengefasst:
Das Kabelführungssystem ermöglicht bei einer vorteilhaft langen translatorischen Verfahrstrecke für die Gantry eine Anwendung in Zwei-Raum-Umgebungen. Das Kabelführungssystem ermöglicht dabei eine 180°-Drehung der Gantry um die Vertikalachse durch ihr Isozentrum. Die Drehung der Gantry verbessert die Zugänglichkeit der Gantry, derart, dass der Patient bzw. eine Patientenliege samt Patient in jedem der Untersuchungsräume einer Zwei-Raum-Umgebung von den Außenseiten der Untersuchungsräume her in die Gantry geschoben werden kann, was die Patientenpositionierung und damit den Untersuchungsablauf stark vereinfacht.

Das Kabelführungssystem benötigt insgesamt weniger Platz, da erfindungsgemäß auf Reserveschlaufen verzichtet werden kann bzw. Reservelängen für die Versorgungsleitung im Inneren des Kabelführungssystems angeordnet sind. Dies schafft vorteilhaft mehr Platz für andere, insbesondere interventionelle Bildgebungssysteme, bspw. ein C-Bogensystem.

Ohne Reserveschlaufen ist das Kabelführungssystem sicherer, denn es umfasst deutlich weniger Gefahrstellen für Verletzungen von Mensch und für Beschädigungen von medizinischer Ausstattung in direkter Umgebung zur CT-Anlage. Der Verzicht auf Reserveschlaufen verringert auch die Gefahr, dass die Versorgungsleitung selbst übermäßig, insbesondere rückwärtig dynamisch verknickt.

Das Kabelführungssystem schafft über das tragende Halteelement eine einfache Zugänglichkeit zu der wenigstens einen Versorgungsleitung. Dies vereinfacht die Wartung des Systems. Zudem ermöglicht das tragende Haltelement eine leichtere Montage des Kabelführungssystems.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine perspektivische Ansicht eines Kabelführungssystems in einer ersten Ausführung der Erfindung,
- FIG 2: eine Draufsicht auf ein Kabelführungssystem in der Ausführung gemäß Figur 1, ,
- FIG 3: eine Detailansicht eines Kabelführungssystems in einer weiteren Ausführung der Erfindung,
- FIG 4: eine weitere Detailansicht des Kabelführungssystems gemäß Figur 3, und
- FIG 5: eine perspektivische Ansicht einer in einer Untersuchungsumgebung angeordneten Computertomographie-Anlage samt Kabelführungssystem in einer Ausführung der Erfindung in einer exemplarischen Betriebsstellung.

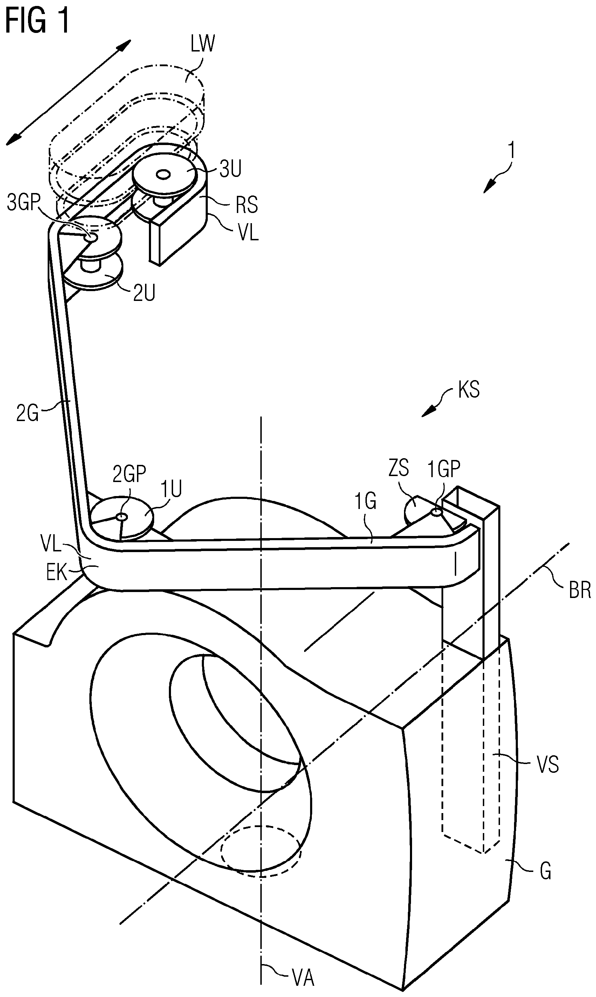

**Figur 1** zeigt eine perspektivische Ansicht eines Kabelführungssystems KS in einer ersten Ausführung der Erfindung. Das Kabelführungssystem KS ist Bestandteil einer Computertomographie-Anlage 1 . Diese weist eine Gantry G auf, die entlang einer zur Gantry G senkrecht verlaufenden Bewegungsrichtung BR verstellt bzw. verschoben werden kann. Das Kabelführungssystem KS umfasst eine hier seitlich bzw. eckständig an der Gantry G angeordnete Vertikalsäule VS, die von einem Sockel der Computertomographie-Anlage 1 vertikal nach oben verläuft. Ferner ist ein erster Gelenkarm 1G sowie ein zweiter Gelenkarm 2G umfasst. Der erste Gelenkarm 1G ist dabei an einem oberen Ende der Vertikalsäule VS oberhalb der Gantry G über einen ersten Gelenkpunkt 1GP rotierbar sowie mit dem ebenfalls oberhalb der Gantry G angeordneten zweiten Gelenkarm 2G über einen zweiten Gelenkpunkt 2GP rotierbar angebunden. Der erste Gelenkpunkt 1GP bewirkt also, dass der erste Gelenkarm 1G nicht nur oberhalb, sondern auch zumindest teilweise über die Gantry G verschwenkt werden kann. Der zweite Gelenkpunkt 2GP bewirkt, dass eine Relativstellung zwischen erstem und zweitem Gelenkarm 1G, 2G verändert werden kann. Die Vertikalsäule VS weist eine Höhe auf, die sich bis über die Gantry G erstreckt. Die Höhe der Vertikalsäule VS ist dabei so ausgelegt, dass ein Minimal- bzw. Sicherheitsabstand zwischen dem höchsten Punkt der Gantry G und der Unterseite des ersten Gelenkarms 1G eingehalten wird. Die Vertikalsäule VS sowie der erste und zweite Gelenkarm 1G, 2G sind jeweils Hohlräume bzw. innenliegende Bereiche aufweisend ausgebildet, sodass sie wenigstens eine, typischerweise eine Vielzahl von Versorgungsleitungen VL in Form von Elektro- oder Datenkabeln zumindest teilweise jeweils entlang ihrer Längsachsen führen können. Mit anderen Worten verlaufen jeweils Teilabschnitte der wenigstens einen Versorgungsleitung VL innerhalb der Vertikalsäule VS, dem ersten Gelenkarm 1G und dem zweiten Gelenkarm 2G. Die wenigstens eine Versorgungsleitung VL erstreckt sich ihrer Länge nach folglich mindestens über die Gesamtlänge von Vertikalsäule VS, erstem und zweitem Gelenkarm 1G, 2G und wird durch den Verlauf der genannten Komponenten des Kabelführungssystems KS geführt.

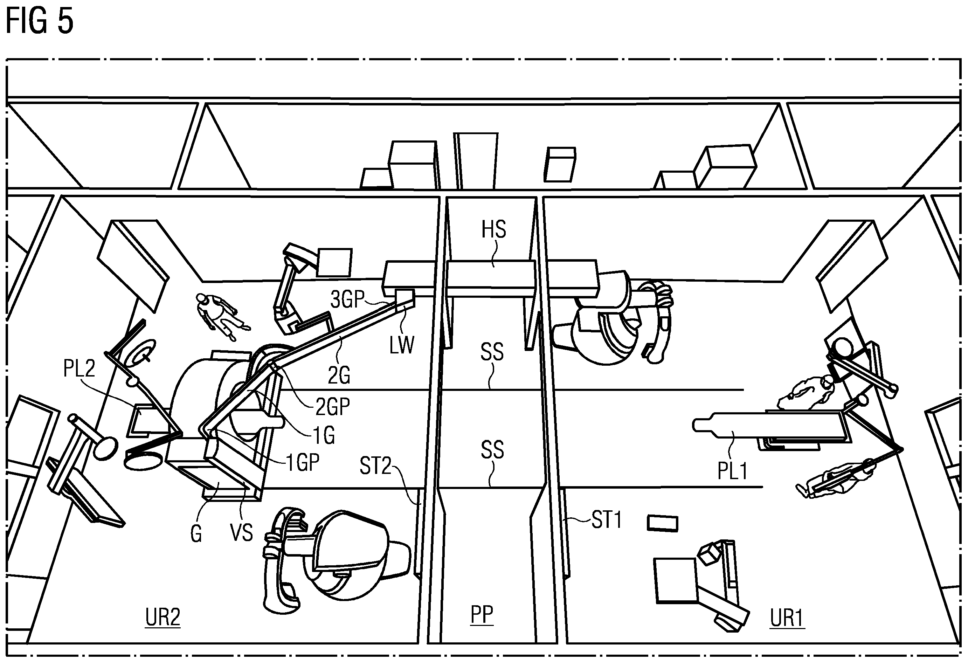

Der zweite Gelenkarm 2G ist in dieser Ausführung weiter über einen deckenständigen dritten Gelenkpunkt 3GP oberhalb der Gantry G rotierbar angebunden. Deckenständig bedeutet in diesem Zusammenhang, dass der dritte Gelenkpunkt 3GP nahe der bzw. an der Decke montiert bzw. befestigt ist. Dadurch kann auch der zweite Gelenkarm 2G nicht nur oberhalb, sondern zumindest auch teilweise über der Gantry G verschwenkt bzw. positioniert werden. Der dritte Gelenkpunkt 3GP ist in dieser Ausführung an einem Laufwagen LW angeordnet. Dieser ist seinerseits an einer oberhalb der Gantry G verlaufenden, deckenständigen, also direkt an der Raumdecke montierten Horizontalsäule HS (vgl. Figur 5) angebracht. Die Horizontalsäule HS verläuft ihrer Längsachse nach parallel zu der Bewegungsrichtung BR der Gantry G. Der Laufwagen LW ist an einer Längsseite der Horizontalsäule HS ihrer Länge nach (vgl. Doppelpfeil) verstellbar angeordnet. Derart kann der Laufwagen LW und damit der dritte Gelenkpunkt 3GP über im Wesentlichen die gesamte Länge der Horizontalsäule HS in Bewegungsrichtung BR der Gantry G mitbewegt werden, was die Bewegungsfreiheit für die Gantry G vorteilhaft erhöht. Die Horizontalsäule HS weist vorliegend eine Länge von 7 m auf. Sie kann aber auch, je nach baulicher Voraussetzung der medizinischen Einrichtung, kürzer ausgeführt werden, bspw. 6 m, 5 m oder dergleichen. In dieser Ausführung der Erfindung umfasst der Laufwagen LW einen Antrieb (nicht gezeigt), der aktiv den Laufwagen LW während einer Verstellbewegung der Gantry G verschiebt. In anderen Ausführungen kann das Verschieben des Laufwagens auch passiv bzw. durch den Antrieb der Gantry G erfolgen.

In weiteren, hier nicht gezeigten Ausführungen ist der dritte Gelenkpunkt 3GP fest an der Raumdecke montiert. Sprich, er lässt sich nicht bewegen. Diese Ausführungen sind besonders gut für Ein-Raum-Anwendungen angepasst.

Der Laufwagen LW ist vorliegend ausgebildet, die Höhe des dritten Gelenkpunktes 3GP zu verstellen. Mit anderen Worten erlaubt der Laufwagen, einen Ausgleich zwischen Raumdeckenhöhe und Höhe der Vertikalsäule VS bzw. den Höhen der drei Gelenkpunkte 1GP, 2GP, 3GP, die immer in einer gemeinsamen Ebene liegen, bevorzugt in einer Horizontalebene, also parallel zur Raumdecke bzw. zum Untergrund. Zu diesem Zweck umfasst der Laufwagen LW in dieser Ausführung einen bspw. teleskopisch ausgebildeten Verstellmechanismus (nicht gezeigt), um durch vertikales Ein- oder Ausfahren von wenigstens einem Teleskop-Segment die Höhe des dritten Gelenkpunktes 3GP einzustellen.

Zusätzlich zu dem Laufwagen LW, der den dritten Gelenkpunkt 3GP in seiner Höhe anpassen kann, ist vorliegend auch die Vertikalsäule VS höhenverstellbar ausgebildet, sodass auch der erste und zweite Gelenkpunkt 1GP, 2GP in ihrer Höhe angepasst werden können, um eine geeignete Betriebsebene für die Gelenkpunkte einzustellen. In diesem Sinne ist in der gezeigten Ausführung zumindest der erste und der dritte Gelenkpunkt 1GP, 3GP ausgebildet, eine Verschwenkbewegung nicht nur um eine Vertikalachse, sondern auch um eine Horizontalachse zu erlauben. Auch die Vertikalsäule VS kann über einen Teleskop-Mechanismus (nicht gezeigt) verfügen, der bevorzugt am oberen Ende der Vertikalsäule VS angeordnet ist, welcher durch Ein- und Ausfahren wenigstens eines Teleskop-Abschnitts die Höhe des ersten Gelenkpunktes 1GP einstellt.

Die wenigstens eine Versorgungsleitung VL verläuft zumindest abschnittsweise in dem Laufwagen LW weiter, wie weiter unten mit Bezug zu den weiteren Figuren noch erläutert wird. Die wenigstens eine Versorgungsleitung VL ist vorliegend vollständig in einer Energiekette EK geführt, also nicht nur über die Länge der beiden Gelenkarme 1G, 2G hinweg, sondern auch in der Vertikalsäule VS sowie in dem Laufwagen.

Die wenigstens eine Versorgungsleitung VL verläuft entlang der Längsachsen des ersten und zweiten Gelenkarms 1G, 2G verstellbar geführt. Das heißt, die Relativposition der Versorgungsleitung VL zu den Gelenkarmen 1G, 2G ist nicht festgelegt, sondern variabel. Die Versorgungsleitung VL ist also zumindest abschnittsweise in dem Kabelführungssystem ihrer Länge nach ggü. weiteren Komponenten des Kabelführungssystems KS verstellbar ausgeführt.

Zu diesem Zweck sind im ersten, zweiten und dritten Gelenkpunkt 1GP, 2GP, 3GP jeweils entweder ein Zylindersegment ZS oder eine erste bzw. eine zweite Umlenkrolle 1U, 2U vorgesehen, um welche die wenigstens eine Versorgungsleitung VL umfänglich geführt ist.

Das heißt, die wenigstens eine Versorgungsleitung VL liegt umfänglich zumindest teilweise an der Mantelfläche des Zylindersegments ZS und der ersten bzw. der zweiten Umlenkrolle 1U, 2U an. Dabei wird die Versorgungsleitung VL umfänglich verstellbar geführt. Das bedeutet, dass die Versorgungsleitung VL entlang ihrer Längsachse verstellbar an den Umlenkrollen 1U, 2U angelagert ist. Der Abschnitt der Versorgungsleitung VL, der an der bzw. um die Umlenkrolle 1U, 2U verläuft, ist entlang der Versorgungsleitungslängsachse variabel. Zudem ist in Abhängigkeit einer Stellung des Kabelführungssystems KS der an einer der Umlenkrolle 1U, 2U und des Zylindersegments ZS anliegende Abschnitt der Versorgungsleitung VL jeweils in seiner Länge variabel.

Insgesamt ist die Länge der Versorgungsleitung VL zwischen dem ersten Gelenkpunkt 1GP (genauer gesagt einem Anbindungspunkt der Versorgungsleitung VL an der Vertikalsäule VS) und dem dritten Gelenkpunkt 3GP genau so lang, dass das Kabelführungssystem KS ohne weitere Leitungsreserve alle beliebigen Stellungen einnehmen kann, die für eine rein translatorische Bewegung der Gantry G der Computertomographie-Anlage 1 entlang der Bewegungsrichtung BR vorgesehen sind. Mit anderen Worten wird allein durch das Zylindersegment ZS sowie die Umlenkrollen 1U, 2U eine für eine translatorische Bewegung der Gantry G ausreichende Versorgungsleitungslänge bereitgestellt. Solange die Gantry G sich nur translatorisch bewegt, ist die Länge der Versorgungsleitung VL zwischen erstem und drittem Gelenkpunkt 1GP, 3GP also konstant.

Für die jeweiligen Abschnitte der Versorgungsleitung VL, die um das Zylindersegment ZS und/oder die Umlenkrollen 1U, 2U verlaufen, bewirkt die Energiekette EK, von der die Versorgungsleitung VL umgeben ist, einen zusätzlichen mechanischen Schutz, denn Energieketten sorgen allgemein für eine Stabilisierung der wenigstens einen Versorgungsleitung, verhindern Beschädigung bzw. übermäßiges Verknicken oder Verdrehen.

Während die Umlenkrollen 1U, 2u rotatorisch in dem zweiten und dritten Gelenkpunkt 2GP, 3GP angeordnet sind, ist das Zylindersegment ZS fest mit der Vertikalsäule verbunden. Das Zylindersegment ZS rotiert folglich nicht bei einer Verstellbewegung der Gantry.

Um eine Rotation der ersten Umlenkrolle 1U und der zweiten Umlenkrolle 2U zu ermöglichen, verläuft die Rotationachse der ersten Umlenkrolle 1U in dieser Ausführung durch den zweiten Gelenkpunkt 2GP und der erste und der zweite Gelenkarm 1G, 2G sind auf der Rotationsachse der ersten Umlenkrolle 1U aneinander angebunden. Entsprechend verläuft die Rotationachse der zweiten Umlenkrolle 2U durch den dritten Gelenkpunkt 3GP und der zweite Gelenkarm 2G ist auf der Rotationsachse der zweiten Umlenkrolle 2U an dem Laufwagen LW angebunden. Derart wird eine relative Beweglichkeit der Gelenkarme 1G, 2G zueinander bzw. zu dem Laufwagen LW und zu den Umlenkrollen 1U, 2U realisiert.

Beim Verschwenken der Gelenkarme 1G, 2G wird die Versorgungsleitung VL relativ zur Gelenkarmlängsachse verstellt bzw. verschoben. Da die Versorgungsleitung VL an den Umlenkrollen 1U, 2U anliegt, werden diese durch die Versorgungsleitung VL in Rotation versetzt und unterstützen so die Verstellbewegung und bewirken ein optimiertes Umlenken der Versorgungsleitung VL. Die seitlichen Außenflächen der Energiekette EK sind vorliegend einerseits auf eine hohe Haftreibung ausgelegt, um die Rotation der Umlenkrollen 1U, 2U bei einer Verstellbewegung zu fördern. Die untere Außenfläche der Energiekette EK ist andererseits auf eine geringe Gleitreibung ausgelegt, um die Verstellbewegung ggü. den Gelenkarmen 1G, 2G zu erleichtern.

Im Gegensatz zu den Umlenkrollen 1U, 2U ist das Zylindersegment ZS starr an der Vertikalsäule VS montiert. Dabei verläuft die Mittelachse des Zylindersegments ZS durch den ersten Gelenkpunkt 1GP und der erste Gelenkarm 1G ist in der Mittelachse an der Vertikalsäule VS angebunden. Derart wird die relative Verstellbarkeit der Versorgungsleitung VL ggü. der Vertikalsäule VS bzw. der Gantry G realisiert.

Der zweite Gelenkpunkt 2GP ist vorliegend derart ausgebildet, dass er Stellungen erlaubt, in denen der erste Gelenkarm und der zweite Gelenkarm 1G, 2G einen Winkel zwischen 10° und 170° einschließen. Erster und dritter Gelenkpunkt 1GP, 3GP können dadurch fast maximal voneinander (entsprechend der Summe der Gelenkarmlängen) entfernt oder minimal voneinander (entsprechend einer Differenz der Gelenkarmlängen) entfernt werden, was die Bewegungsfreiheit für die Gantry G weiter erhöht.

Der dritte Gelenkpunkt 3GP ist ausgebildet, dass er Stellungen erlaubt, in denen der zweite Gelenkarm 2G und Horizontalsäulenlängsachse einen Winkel zwischen 10° und 270° einschließen. Dadurch wird die Bewegungsfreiheit der CT-Anlage 1 weiter erhöht, da somit auch der zweite Gelenkarm 2G ausgehend vom dritten Gelenkpunkt 3 GP einen breiten Winkelbereich überspannen kann.

Der erste Gelenkpunkt 1GP ist weiter ausgebildet ist, dass er Stellungen erlaubt, in denen der erste Gelenkarm 1G und die Gantry G einen Winkel zwischen 10° und 180° einschließen.

Das Gelenksystem umfassend die beiden Gelenkarme 1G, 2G und die drei Gelenkpunkte 1GP, 2GP, 3GP erlaubt aufgrund der großen Winkelbereiche an den verschiedenen Gelenkpunkten beliebige Stellungen der Gantry G. Durch das Vorsehen des Laufwagens LW bzw. der Horizontalsäule HS wird der Bewegungsradius für die Gantry G weiter vorteilhaft erweitert. Mittels der besonders breit vorgegebenen Rotationswinkelbereiche des ersten und des zweiten Gelenkpunktes 1GP, 2GP ermöglicht das Kabelführungssystem KS insbesondere eine Rotation der Gantry G um 180° und darüber hinaus.

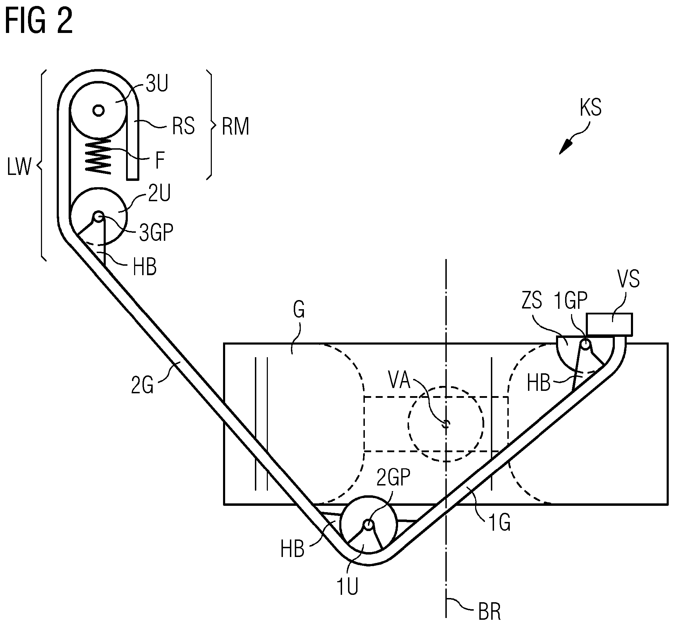

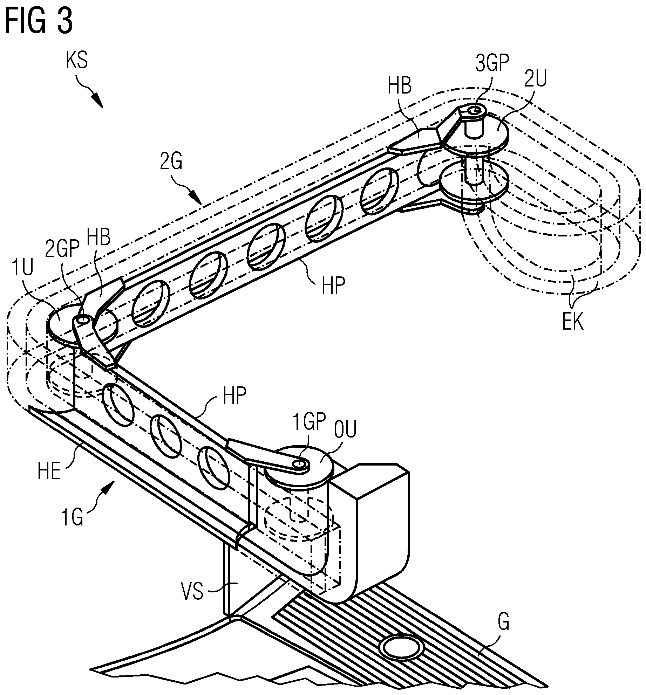

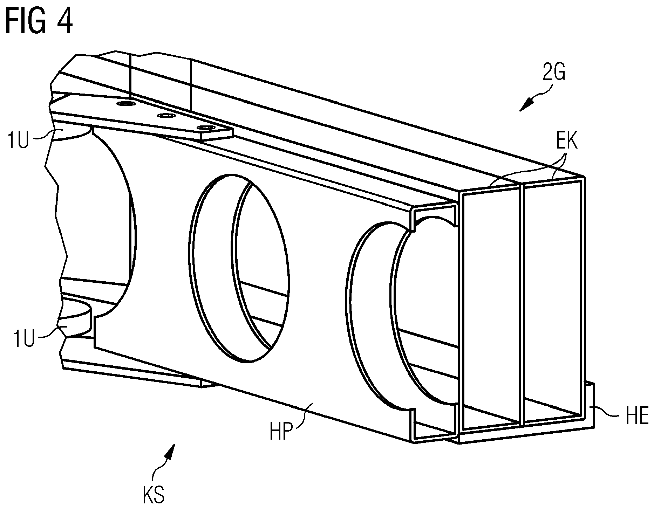

Zur Realisierung dieser großen Winkelbereiche sind an den Gelenkarmen 1G, 2G endständig jeweils zwei Hebelarme HB vorgesehen, die sich in einem Winkel zwischen 30° und 120° von den Gelenkarmen weg erstrecken und zumindest teilweise die effektive Länge eines Gelenkarms erhöhen. Die Hebelarme HB weisen eine Länge auf, die mindestens dem Radius der Umlenkrollen 1U, 2U oder des Zylindersegments ZS entspricht. Die Hebelarme HB können einstückig an die Gelenkarme 1G, 2G angeformt sein, so wie in den Figuren 1 und 2 gezeigt. Alternativ können die Hebelarme HB auch einzeln gefertigt und anschließend an die Gelenkarme bspw. mittels Schrauben oder Bolzen angefügt werden, wie in den Figuren 3 und 4 veranschaulicht.

Die Länge des ersten Gelenkarms 1G entspricht erfindungsgemäß 65% bis 75%, hier ca. 70% der Länge des zweiten Gelenkarms 2G. Durch Einhaltung dieses Längenverhältnisses kann die Gantry G ortsfest um eine Vertikalachse VA um 180° gedreht werden. Vorliegend erfordern die Dimensionen der Gantry G sowie der Einsatz einer Computertomographie-Anlage 1 in einer Zwei-Raum-Umgebung, dass der erste Gelenkarm 1G 1600 mm und der zweite Gelenkarm 2G 2300 mm lang ist.

Ohne Horizontalsäule HS bzw. Laufwagen LW ermöglicht das Kabelführungssystem KS mit diesen Gelenkarmlängen eine maximale Verfahrstrecke entlang der Bewegungsrichtung BR für die Gantry G von 5600 mm, mit 7 m langer Horizontalsäule HS erhöht sich die maximale Verfahrstrecke auf 12 m.

**Figur 2** zeigt eine Draufsicht auf ein Kabelführungssystem in der Ausführung gemäß Figur 1.

Wie eingangs bereits erläutert, ist in dem Laufwagen LW ein Reservemodul RM angeordnet, welches eine Versorgungsleitungsreserve in Form einer zusätzlichen Reserveschlaufe RS bereitstellt, um eine ausreichende Versorgungsleitungslänge für eine Rotation der Gantry G um ihre Vertikalachse VA, insbesondere um 180° bereit zu stellen. Das Reservemodul RM umfasst in dieser Ausführung eine dritte Umlenkrolle 3U, um welche die Reserveschlaufe RS umfänglich verstellbar geführt verläuft. Die umfänglich verstellbare Führung ist dabei in dem Sinne der umfänglich verstellbaren Führung zu verstehen, wie sie bereits mit Bezug zu der ersten und zweiten Umlenkrolle 1U, 2U weiter oben beschrieben wurde. Während das Ende der Reserveschlaufe RS an einem Fixpunkt im Laufwagen LW angebunden ist, ist die dritte Umlenkrolle 3U gegen die Federkraft einer Feder F verstellbar gelagert. Bei einer Rotation der Gantry G um ihre Vertikalachse VA übt die Versorgungsleitung VL eine Zugkraft auf das Reservemodul RM aus, wobei die dritte Umlenkrolle 3U gegen die Federkraft in Zugkraftrichtung verstellt wird, wodurch die Reserveschlaufe zumindest teilweise freigegeben bzw. in den Bereich des zweiten Gelenkarms 2G verschoben wird.

**Figur 3** zeigt eine Detailansicht eines Kabelführungssystems KS in einer weiteren Ausführung der Erfindung. Neben der ggü. der unterschiedlichen Ausgestaltung der Hebelarme HB im Vergleich zu der in den Figuren 1 und 2 gezeigten Ausführung des Kabelführungssystems KS, ist hier anstelle eines Zylindersegments ZS im ersten Gelenkpunkt 1GP ein weiteres Umlenkelement 0U vorgesehen, über welches die Versorgungsleitung VL zwischen ihrem Anbindungspunkt an der Vertikalsäule VS und dem ersten Gelenkarm 1G verstellbar geführt verläuft. Der erste und der zweite Gelenkarm 1G, 2G sind vorliegend als Stahlblech-Hohlprofilteile HP ausgebildet, die einen im Wesentlichen rechteckeigen Querschnitt aufweisen. Die Seiten des Hohlprofils sind vorliegend mit einer Mehrzahl von kreisrunden Ausnehmungen ausgebildet, um das Gewicht der Gelenkarme 1G, 2G positiv zu senken.

**Figur 4** zeigt eine weitere Detailansicht des Kabelführungssystems KS gemäß Figur 3. Aus beiden Figuren lässt sich der Verlauf eines Dachrinnen-förmigen Haltelements HE insbesondere in Bezug zum zweiten Gelenkarm 2G erkennen, welches sich aber im Wesentlichen über die gesamte Länge eines jeden Gelenkarms an einer der Außenseiten des Hohlprofils HP erstreckt. So wie die Hebelarme HB sind in dieser Ausführung auch die Halteelemente HE nachträglich an das Hohlprofil HP eines jeden Gelenkarms 1G, 2G angefügt. Andere Ausgestaltungen sind natürlich denkbar. Das Halteelement HE ist ebenfalls als Stahlblechbauteil ausgeführt und es ist als tragende Struktur ausgebildet. Dies ermöglicht es, während der Montage oder während einer Wartung des Kabelführungssystems KS die wenigstens eine Energiekette EK samt jeweils wenigstens einer Versorgungsleitung VL in dem Halteelement HE abzulegen. Die Energiekette EK muss nun nicht mehr über einen längeren Zeitraum von Montagepersonal in Position gehalten werden, sondern wird dort einmalig positioniert und dann in dem Halteelement HE gehalten.

Das Halteelement HE kann dann mittels einer nicht tragenden und wieder entfernbaren Abdeckung nach außen hin im Wesentlichen vollständig verschlossen werden, um die Energiekette EK im Inneren der Gelenkarme 1G, 2G zu schützen.

Ebenfalls in den Figuren 4 und 5 gezeigt ist, dass zwei aufrecht ausgerichtete Energieketten EK parallel zueinander in den Halteelementen HE angeordnet und geführt werden.

**Figur 5** zeigt eine perspektivische Ansicht einer in einer Untersuchungsumgebung angeordneten Computertomographie(CT)-Anlage 1 samt Kabelführungssystem KS in einer Ausführung der Erfindung in einer exemplarischen Betriebsstellung. Die Untersuchungsumgebung ist eine Zwei-Raum-Umgebung umfassend zwei Untersuchungsräume UR1 und UR2. Diese werden von einer Parkposition PP für die CT-Anlage 1 räumlich voneinander getrennt. Wird die CT-Anlage 1 nicht gebraucht bzw. ist sie außer Betrieb, kann sie in die Parkposition PP verbracht und die Untersuchungsräume UR1, UR2 weiter für andere medizinische Anwendungen genutzt werden. Zu diesem Zweck sind in den Untersuchungsräumen UR1, UR2 jeweils auch weitere medizinische Geräte und Anlagen, unter anderem C-Bögen oder Monitore zur in-situ Bildanzeige oder Geräte zur Überwachung physiologischer Funktionen eines Patienten vorgesehen. Des Weiteren sind in jedem Untersuchungsraum UR1, UR2 jeweils Patientenliegen PL1, PL2 angeordnet, die jeweils fest mit dem Boden verbunden, also ortsfest angeordnet sind. Die Liegenbretter sind gegenüber einem Liegensockel verstellbar, insbesondere translatorisch verstellbar, um eine Feinpositionierung des Patienten für eine Bildgebung und/oder einen interventionellen Eingriff zu ermöglichen.

Die Computertomographie-Anlage 1 dient zur Erzeugung tomographischer Röntgenbilder, sowohl in dem ersten als auch in dem zweiten Untersuchungsraum UR1, UR2. Entsprechend umfasst sie eine Gantry G, die in einer zur Gantry G senkrecht verlaufenden Bewegungsrichtung BR verstellbar ist. Die Bewegungsrichtung BR erstreckt sich hier entlang des Schienensystems SS umfassend zwei Führungsschienen. Die Schienen weisen, wie weiter oben bereits beschrieben, eine Länge von 12 m auf. Ihre Länge und ihr Verlauf definieren den Bewegungsradius der Gantry G.

Die Gantry G ist ferner um eine durch das Isozentrum der Computertomographie-Anlage 1 verlaufende Vertikalachse VA rotierbar ausgebildet. Dies wird durch das oben bereits beschriebene Kabelführungssystem KS umfassend mehrere Komponenten ermöglicht, welches ebenfalls von der CT-Anlage 1 umfasst ist. Insbesondere umfasst das Kabelführungssystem KS eine Horizontalsäule HS mit 7 m Länge, die derart angeordnet ist, dass sie die Parkposition PP überragt und in beide Untersuchungsräume UR1, UR2 hineinragt. Dabei kann eine Rotation ortsfest, also ohne parallele Translation der Gantry G entlang des Schienensystems SS bzw. des Laufwagens LW in paralleler Richtung allein durch Verstellen der Gelenkarme 1G, 2G erfolgen. Alternativ kann sie zusammen mit bzw. kontinuierlich parallel zu einer translatorischen Bewegung der Gantry G entlang des Schienensystems SS erfolgen.

In Figur 5 steht die Gantry G im Untersuchungsraum UR2 in der linken Maximalstellung entsprechend des linken Endes des Schienensystems SS. Die Gantry G ist derart rotiert, dass sie mit ihrer Frontseite auf die Außenseite (links) des Untersuchungsraums 2 gerichtet ist. In dieser Stellung ragt die Patientenliege PL2 mit ihrem Liegenbrett in die Bore der Gantry G hinein. In dieser Stellung der Gantry G können tomographische Röntgenbilder eines Patienten erzeugt werden. Um diese Stellung zu erreichen, nimmt das Kabelführungssystem KS eine maximal gestreckte Stellung ein. Gelenkpunkt 2GP ist auf ca. 170° aufgeweitet, sodass erster und zweiter Gelenkarm 1G, 2G im Wesentlichen hintereinander liegen und sich ihre Längen aufaddieren. Beide Gelenkarme 1G, 2G verlaufen nicht über der Gantry G. Der Laufwagen LW befindet sich ebenfalls in seiner linken Maximalstellung in Bezug auf die Horizontalsäule HS. Dennoch sind der dritte und der erste Gelenkpunkt 1GP, 3GP maximal beabstandet. Derart kann über das Kabelführungssystem KS die erforderliche Länge der Versorgungsleitung bis zu einem Anschlusspunkt am unteren Ende der Vertikalsäule VS bereitgestellt werden.

Die CT-Anlage 1 ist nun aufgrund der Ausbildung des Kabelführungssystems KS ausgebildet, von der linken Maximalstellung in die rechte Maximalstellung im gegenüberliegenden Untersuchungsraum UR1 überzugehen, wobei verschiedene Zwischenstellungen, insbesondere eine Stellung in der Parkposition PP eingenommen werden können. In der rechten Maximalstellung ist die Gantry G gegenüber der Figur 5 um 180°, also vollständig rotiert und sie ist mit ihrer Frontseite auf die Außenseite (rechts) des Untersuchungsraums UR1 gerichtet. In dieser Stellung ragt die Patientenliege PL1 mit ihrem Liegenbrett in die Bore der Gantry G hinein. In dieser Stellung der Gantry G können wieder tomographische Röntgenbilder eines Patienten erzeugt werden. Insbesondere bewirkt die Drehung, dass die Patientenliege in jedem Untersuchungsraum von der Raumaußenseite her in die Bore eingeführt werden und damit umständliche Verstellbewegungen mit der Patientenliege zum Patientenwohl und zur Vereinfachung des Untersuchungsablaufs vermieden werden können. Zudem können die Patientenliegen PL1, PL2 fest in den Untersuchungsräumen UR1, UR2 installiert werden. Um die rechte Maximalstellung zu erreichen, nimmt das Kabelführungssystem KS eine gestreckte, wenn auch nicht maximal gestreckte Stellung ein. Gelenkpunkt 2GP ist auf ca. 105° aufgeweitet, sodass sich die Längen des ersten und zweiten Gelenkarms 1G, 2G weitgehend aufaddieren. Beide Gelenkarme 1G, 2G verlaufen wieder nicht über der Gantry G. Der Laufwagen LW befindet sich in seiner rechten Maximalstellung in Bezug auf die Horizontalsäule HS. Der dritte und der erste Gelenkpunkt 1GP, 3GP sind nicht maximal, aber dennoch sehr weit beabstandet. Auch in dieser Stellung der Gantry G kann das Kabelführungssystem KS die erforderliche Länge der Versorgungsleitung bis zu dem Anschlusspunkt am unteren Ende der Vertikalsäule VS bereitstellen.

Auf dem Weg von der linken Maximalstellung hin zur rechten Maximalstellung kann die CT-Anlage 1 auch eine Parkstellung (nicht gezeigt) durchlaufen, in welcher die CT-Anlage 1 außer Betrieb ist. Die CT-Anlage 1 nimmt die Parkstellung innerhalb der Parkposition PP ein. Bei der Verstellbewegung der Gantry G geöffnete Schiebetüren ST1, ST2 der Parkposition PP können dabei geschlossen werden, sodass jeder Untersuchungsraum UR1, UR2 unabhängig voneinander anderweitig genutzt werden kann.

Die Gantry G nimmt in der Parkstellung eine vorab definierte, maximal rechts in der Parkposition PP liegende Position ein, wobei sie entsprechend der rechten Maximalposition derart rotiert ist, dass ihre Front auf die Außenseite (rechts) von Untersuchungsraum UR1 ausgerichtet ist. Der zweite Gelenkpunkt GP2 schließt hier einen Winkel von 10° zwischen den beiden Gelenkarmen 1G, 2G ein, sodass diese platzsparend im Wesentlichen nebeneinander angeordnet sind. Insbesondere der zweite Gelenkarm 2G verläuft in dieser Stellung im Wesentlichen parallel zur Gantry-Rückseite. Zum Erreichen dieser Parkstellung wird der Laufwagen LW mittels seines Antriebs aktiv an die entsprechende Position entlang seines Verstellwegs an der Horizontalsäule HS bewegt, um den Winkel von 10° im zweiten Gelenkpunkt 2GP zu erreichen.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Computertomographie-Anlage (1) zur Erzeugung tomographischer Röntgenbilder, umfassend eine Gantry (G), die in einer zur Gantry (G) senkrecht verlaufenden Bewegungsrichtung (BS) verstellbar ausgebildet ist, sowie ein Kabelführungssystem (KS) umfassend
- eine an der Gantry (G) angeordnete Vertikalsäule (VS), die von einem Sockel der Computertomographie-Anlage (1) vertikal nach oben verläuft, und
- einen ersten Gelenkarm (1G) sowie einen zweiten Gelenkarm (2G), wobei der erste Gelenkarm (1G) an einem oberen Ende der Vertikalsäule (VS) oberhalb der Gantry (G) über einen ersten Gelenkpunkt (1GP) rotierbar sowie mit dem ebenfalls oberhalb der Gantry (G) angeordneten zweiten Gelenkarm (2G) über einen zweiten Gelenkpunkt (2GP) rotierbar angebunden ist,
wobei
wenigstens eine Versorgungsleitung (VL) entlang der Längsachsen des ersten und des zweiten Gelenkarms (1G, 2G) verstellbar geführt verläuft.

2. Computertomographie-Anlage (1) nach Anspruch 1, wobei an dem ersten und/oder dem zweiten Gelenkarm (1G, 2G) eine erste und/oder eine zweite Umlenkrolle (1U, 2U) vorgesehen ist, um welche die Versorgungsleitung (VL) umfänglich verstellbar geführt verläuft.

3. Computertomographie-Anlage (1) nach Anspruch 1 oder 2, bei dem in dem ersten Gelenkpunkt (1GP) ein Zylindersegment (ZS) angeordnet ist, um welches die Versorgungsleitung (VL) umfänglich verstellbar geführt verläuft.

4. Computertomographie-Anlage (1) nach einem der vorherigen Ansprüche, wobei die erste Umlenkrolle (1U) an dem zweiten Gelenkpunkt (2GP) angeordnet ist.

5. Computertomographie-Anlage (1) nach einem der Ansprüche 2 bis 4, wobei der zweite Gelenkarm (2G) weiter über einen deckenständigen dritten Gelenkpunkt (3GP) oberhalb der Gantry (G) rotierbar angebunden ist, in welchem die zweite Umlenkrolle (2U) angeordnet ist, um die die Versorgungsleitung (VL) umfänglich verstellbar geführt verläuft.

6. Computertomographie-Anlage (1) nach einem der vorherigen Ansprüche, wobei die wenigstens eine Versorgungsleitung (VL) in einer Energiekette (EK) angeordnet ist.

7. Computertomographie-Anlage (1) nach einem der vorherigen Ansprüche, wobei der zweite Gelenkpunkt (2GP) derart ausgebildet ist, dass er Stellungen erlaubt, in denen der erste Gelenkarm (1G) und der zweite Gelenkarm (2G) einen Winkel zwischen 10° und 170° einschließen.

8. Computertomographie-Anlage (1) nach Anspruch 4, wobei die Rotationachse der ersten Umlenkrolle (1U) durch den zweiten Gelenkpunkt (2GP) verläuft und der erste und der zweite Gelenkarm (1G, 2G) auf der Rotationsachse der ersten Umlenkrolle (1U) aneinander angebunden sind.

9. Computertomographie-Anlage (1) nach Anspruch 3, wobei die Mittelachse des Zylindersegments (ZS) durch den ersten Gelenkpunkt (1GP) verläuft und der erste Gelenkarm (1G) in der Mittelachse an der Vertikalsäule (VS) angebunden ist.

10. Computertomographie-Anlage (1) nach einem der vorherigen Ansprüche, ferner umfassend eine oberhalb der Gantry (G) verlaufende, deckenständige Horizontalsäule (HS), die sich in ihrer Längsachse parallel zu der Bewegungsrichtung (BR) der Gantry (G) erstreckt, wobei an der Horizontalsäule (HS) ein in Längsrichtung der Horizontalsäule (HS) verstellbarer Laufwagen (LW) angeordnet ist, der den dritten Gelenkpunkt (3GP) trägt.

11. Computertomographie-Anlage (1) nach Anspruch 10, wobei der dritte Gelenkpunkt (3GP) derart ausgebildet ist, dass er Stellungen erlaubt, in denen der zweite Gelenkarm (2GP) und der Laufwagen (LW) einen Winkel zwischen 10° und 270° einschließen.

12. Computertomographie-Anlage (1) nach einem der Ansprüche 9 bis 11, wobei die Rotationachse der zweiten Umlenkrolle (2U) durch den dritten Gelenkpunkt (3GP) verläuft und der zweite Gelenkarm (2G) auf der Rotationsachse der zweiten Umlenkrolle (2U) an dem Laufwagen (LW) angebunden ist.

13. Computertomographie-Anlage (1) nach einem der Ansprüche 9 bis 12, wobei der Laufwagen (LW) ein Reservemodul (RM) zur Bereitstellung einer Versorgungsleitungsreserve (RS) umfasst.

14. Computertomographie-Anlage (1) nach Anspruch 13, wobei das Reservemodul (RM) eine Versorgungsleitungsschlaufe (RS), sowie eine dritte gegen eine Federkraft angestellte Umlenkrolle (3U) umfasst, um welche die Versorgungsleitungsschlaufe (RS) umfänglich verstellbar geführt ist.

15. Computertomographie-Anlage (1) nach einem der vorherigen Ansprüche, wobei der erste und der zweite Gelenkarm (1G, 2G) jeweils als tragendes Hohlprofil (HP) ausgebildet sind, wobei die wenigstens eine Versorgungsleitung (VL) entlang der Längsachsen des ersten und des zweiten Gelenkarms (1G, 2G) außerhalb des Hohlprofils (HP) in einem Halteelement (HE) verläuft.

16. Computertomographie-Anlage (1) zur Erzeugung tomographischer Röntgenbilder nach einem der vorherigen Ansprüche, umfassend ein Schienensystem (SS), auf welchem die Gantry (G) über eine Strecke von maximal 12 m zwischen zwei Untersuchungsräumen (UR1, UR2) verstellbar ist.

17. Computertomographie-Anlage (1) zur Erzeugung tomographischer Röntgenbilder nach einem der vorherigen Ansprüche, wobei die Gantry (G) ausgebildet ist, bei einer Verstellbewegung entlang des Schienensystems (SS) eine Rotation um eine durch das Isozentrum der Gantry (G) verlaufende Vertikalachse (VA) um 180° zu vollziehen.

## Claims

1. Computed tomography system (1) for generating tomographic X-ray images, comprising a gantry (G), which is configured to be repositionable in a direction of movement (BS) running perpendicular to the gantry (G), as well as a cable guidance system (KS) comprising
- a vertical column (VS) which is arranged on the gantry (G) and runs vertically upward from a base of the computed tomography system (1), and
- a first articulated arm (1G) and a second articulated arm (2G), wherein the first articulated arm (1G) is rotatably connected to an upper end of the vertical column (VS) above the gantry (G) via a first point of articulation (1GP) and rotatably connected via a second point of articulation (2GP) to the second articulated arm (2G) which is likewise arranged above the gantry (G),
wherein
at least one supply line (VL) runs in a repositionably guided manner along the longitudinal axes of the first and second articulated arms (1G, 2G).

2. Computed tomography system (1) according to claim 1, wherein a first and/or a second deflection roller (1U, 2U), about which the supply line (VL) runs in a circumferentially repositionably guided manner, is provided on the first and/or the second articulated arm (1G, 2G).

3. Computed tomography system (1) according to claim 1 or 2, in which a cylinder segment (ZS), about which the supply line (VL) runs in a circumferentially repositionably guided manner, is arranged in the first point of articulation (1GP).

4. Computed tomography system (1) according to one of the preceding claims, wherein the first deflection roller (1U) is arranged at the second point of articulation (2GP).

5. Computed tomography system (1) according to one of claims 2 to 4, wherein the second articulated arm (2G) is further rotatably connected via a ceiling-mounted third point of articulation (3GP) above the gantry (G), in which is arranged the second deflection roller (2U), about which the supply line (VL) runs in a circumferentially repositionably guided manner.

6. Computed tomography system (1) according to one of the preceding claims, wherein the at least one supply line (VL) is arranged in an energy chain (EK).

7. Computed tomography system (1) according to one of the preceding claims, wherein the second point of articulation (2GP) is configured such that it permits positions in which the first articulated arm (1G) and the second articulated arm (2G) form an angle of between 10° and 170°.

8. Computed tomography system (1) according to claim 4, wherein the axis of rotation of the first deflection roller (1U) runs through the second point of articulation (2GP) and the first and second articulated arms (1G, 2G) are connected together on the axis of rotation of the first deflection roller (1U).

9. Computed tomography system (1) according to claim 3, wherein the centre axis of the cylinder segment (ZS) runs through the first point of articulation (1GP) and the first articulated arm (1G) is connected to the vertical column (VS) in the centre axis.

10. Computed tomography system (1) according to one of the preceding claims, further comprising a ceiling-mounted horizontal column (HS) extending above the gantry (G), the longitudinal axis of which column extends parallel to the direction of movement (BR) of the gantry (G), wherein a carriage (LW) which is repositionable in the longitudinal direction of the horizontal column (HS) and bears the third point of articulation (3GP) is arranged on the horizontal column (HS).

11. Computed tomography system (1) according to claim 10, wherein the third point of articulation (3GP) is configured such that it permits positions in which the second articulated arm (2G) and the carriage (LW) form an angle of between 10° and 270°.

12. Computed tomography system (1) according to one of claims 9 to 11, wherein the axis of rotation of the second deflection roller (2U) runs through the third point of articulation (3GP) and the second articulated arm (2G) is connected to the carriage (LW) on the axis of rotation of second deflection roller (2U).

13. Computed tomography system (1) according to one of claims 9 to 12, wherein the carriage (LW) comprises a reserve module (RM) for providing a reserve of supply line (RS).

14. Computed tomography system (1) according to claim 13, wherein the reserve module (RM) comprises a loop (RS) of supply line and a third deflection roller (3U) arranged against a spring force about which the loop (RS) of supply line is circumferentially repositionably guided.

15. Computed tomography system (1) according to one of the preceding claims, wherein the first and the second articulated arms (1G, 2G) each take the form of a load-bearing hollow profile (HP), wherein the at least one supply line (VL) runs in a mounting element (HE) along the longitudinal axes of the first and second articulated arms (1G, 2G) outside the hollow profile (HP).

16. Computed tomography system (1) for generating tomographic X-ray images according to one of the preceding claims, comprising a rail system (SS), on which the gantry (G) is repositionable between two examination rooms (UR1, UR2) over a distance of at most 12 m.

17. Computed tomography system (1) for generating tomographic X-ray images according to one of the preceding claims, wherein the gantry (G) is configured to perform a rotation of 180° about a vertical axis (VA) running through the isocentre of the gantry (G) during a repositioning movement along the rail system (SS).

## Revendications

1. Installation (1) de tomodensitométrie assistée par ordinateur de production d'images de rayons X de tomodensitométrie, comprenant un portique (G), qui est constitué de manière à pouvoir se déplacer dans une direction (BS) de déplacement s'étendant perpendiculairement au portique (G), ainsi qu'un système (KS) de guide-câble, comprenant
- une colonne (VS) verticale, qui est montée sur le portique (G) et qui s'étend vers le haut verticalement à partir d'un socle de l'installation (1) de tomodensitométrie assistée par ordinateur, et
- un premier bras (1G) articulé ainsi qu'un deuxième bras (2G) articulé, dans laquelle le premier bras (1G) articulé est articulé tournant à une extrémité supérieure de la colonne (VS) verticale au-dessus du portique (G) par un premier point (1GP) d'articulation, ainsi qu'est articulé tournant, par un deuxième point (2GP) d'articulation, au deuxième bras (2G) articulé également au-dessus du portique (G),
dans laquelle
au moins une ligne (VL) d'alimentation s'étend, en étant guidée de manière à pouvoir se déplacer, le long des axes longitudinaux du premier et du deuxième bras (1G, 2G) articulés.

2. Installation (1) de tomodensitométrie assistée par ordinateur suivant la revendication 1, dans laquelle il est prévu, sur le premier et/ou le deuxième bras (1G, 2G) articulés, une première et/ou une deuxième poulie (1U, 2U) de déviation, autour de laquelle la ligne (VL) d'alimentation est guidée de manière à pouvoir se déplacer sur le pourtour.

3. Installation (1) de tomodensitométrie assistée par ordinateur suivant la revendication 1 ou 2, dans laquelle, au premier point (1GP) d'articulation, est disposé un segment (ZS) de cylindre, autour duquel la ligne (VL) d'alimentation est guidée de manière à pouvoir se déplacer sur le pourtour.

4. Installation (1) de tomodensitométrie assistée par ordinateur suivant l'une des revendications précédentes, dans laquelle la première poulie (1U) de déviation est montée au deuxième point (2GP) d'articulation.

5. Installation (1) de tomodensitométrie assistée par ordinateur suivant l'une des revendications 2 à 4, dans laquelle le deuxième bras (2G) articulé est monté à rotation au-dessus du portique (G) en outre par un troisième point (3GP) d'articulation, où la deuxième poulie (2U) de déviation est montée, sur le pourtour de laquelle la ligne (VL) d'alimentation s'étend en étant guidée de manière à pouvoir se déplacer.

6. Installation (1) de tomodensitométrie assistée par ordinateur suivant l'une des revendications précédentes, dans laquelle la au moins une ligne (VL) d'alimentation est montée dans une chaîne (EK) d'énergie.

7. Installation (1) de tomodensitométrie assistée par ordinateur suivant l'une des revendications précédentes, dans laquelle le deuxième point (2GP) d'articulation est constitué de manière à permettre des positions, dans lesquelles le premier bras (1G) articulé et le deuxième bras (2G) articulé font un angle compris entre 10° et 170°.

8. Installation (1) de tomodensitométrie assistée par ordinateur suivant la revendication 4, dans laquelle l'axe de rotation de la première poulie (1U) de déviation passe par le deuxième point (2GP) d'articulation et le premier et le deuxième bras (1G, 2G) articulés sont articulés l'un à l'autre sur l'axe de rotation de la première poulie (1U) de déviation.

9. Installation (1) de tomodensitométrie assistée par ordinateur suivant la revendication 3, dans laquelle l'axe médian du segment (ZS) de cylindre passe par le premier point (1GP) d'articulation et le premier bras (1G) articulé est articulé dans l'axe médian de la colonne (VS) verticale.

10. Installation (1) de tomodensitométrie assistée par ordinateur suivant l'une des revendications précédentes, comprenant en outre une colonne (HS) horizontale au plafond s'étendant au-dessus du portique (G), qui s'étend dans son axe longitudinal parallèlement à la direction (BR) de déplacement du portique (G), dans laquelle, sur la colonne (HS) horizontale, est monté un chariot (LW) pouvant se déplacer dans la direction longitudinale de la colonne (HS) horizontale et portant le troisième point (3GP) d'articulation.

11. Installation (1) de tomodensitométrie assistée par ordinateur suivant la revendication 10, dans laquelle le troisième point (3GP) d'articulation est constitué de manière à permettre des positions, dans lesquelles le deuxième bras (2GP) articulé et le chariot (LW) font un angle compris entre 10° et 270°.

12. Installation (1) de tomodensitométrie assistée par ordinateur suivant l'une des revendications 9 à 11, dans laquelle l'axe de rotation de la deuxième poulie (2U) de déviation passe par le troisième point (3GP) d'articulation et le deuxième bras (2G) articulé est articulé au chariot (LW) sur l'axe de rotation de la deuxième poulie (2U) de déviation.

13. Installation (1) de tomodensitométrie assistée par ordinateur suivant l'une des revendications 9 à 12, dans laquelle le chariot (LW) comprend un module (RM) de réserve pour disposer d'une réserve (RS) de ligne d'alimentation.

14. Installation (1) de tomodensitométrie assistée par ordinateur suivant la revendication 13, dans laquelle le module (RM) de réserve comprend une boucle (RS) de ligne d'alimentation, ainsi qu'une troisième poulie (3U) de déviation, autour de laquelle la boucle (RS) de ligne d'alimentation est guidée de manière à pouvoir se déplacer sur le pourtour.

15. Installation (1) de tomodensitométrie assistée par ordinateur suivant l'une des revendications précédentes, dans laquelle le premier et le deuxième bras (1G, 2G) articulés sont constitués respectivement sous la forme d'un profilé creux (HP) porteur, dans laquelle la au moins une ligne (VL) d'alimentation s'étend dans un élément (HE) de maintien, le long des axes longitudinaux du premier et du deuxième bras (1G, 2G) articulés à l'extérieur du profilé (HP) creux.

16. Installation (1) de tomodensitométrie assistée par ordinateur pour la production d'images de rayons X de tomodensitométrie suivant l'une des revendications précédentes, comprenant un système (SS) de rails, sur lequel le portique (G) peut être déplacé sur une distance de 12 m au maximum entre deux espaces (UR1, UR2) d'examen.

17. Installation (1) de tomodensitométrie assistée par ordinateur de production d'images de rayons X de tomodensitométrie suivant l'une des revendications précédentes, dans laquelle le portique (G) est constitué pour, lors d'un mouvement de déplacement le long du système (SS) de rails, effectuer une rotation de 180° autour d'un axe (VA) vertical passant par l'isocentre du portique (G).
